# EUROPÄISCHE PATENTSCHRIFT

(11) **EP 0 678 502 B1**
(45) Veröffentlichungstag und Bekanntmachung des Hinweises auf die Patenterteilung: **31.03.1999**
(21) Anmeldenummer: 95105178.8
(22) Anmeldetag: 06.04.1995
(51) Int. Cl.: C07C 235/16, C07C 235/68

(54) **Verfahren zur Herstellung von Glykoloylaniliden**
Process for preparing glycoloylanilides
Procédé pour la préparation de glycoloylanilides

(30) Priorität: 19.04.1994 DE 4413618
(43) Veröffentlichungstag der Anmeldung: 25.10.1995
(73) Patentinhaber: Clariant GmbH, 65929 Frankfurt am Main (DE)
(72) Erfinder: Planker, Siegfried, Dr., D-61462 Königstein (DE); Papenfuhs, Theodor, Dr., D-60433 Frankfurt am Main (DE)

(56) Entgegenhaltungen:
- EP-A- 0 221 494
- EP-A- 0 300 344
- DE-A- 2 904 490
- DE-A- 3 244 956
- CHEMICAL ABSTRACTS, Band 91, Nr. 3, 16. Juli 1979, Columbus, Ohio, USA Y. INUKAI et al. "Ortho- -Disubstituted F-benzenes (perfluorobenzenes). I. Preparation of (F-benzo)heterocyclic compounds from F-aniline and the reactions of some intermediate (F-phenyl)amino compounds" Seite 648, Nr. 20 418z; & Bull.Chem.Soc.Jpn. 1979, 52(2), 516-20 (Eng.)
- CHEMICAL ABSTRACTS, Band 94, Nr. 11, 16. MUrz 1981, Columbus, Ohio, USA SHIONOGI AND CO. "Imidazoles" Seite 728, Nr. 84 116u; & JP-A-80 094 371

## Beschreibung

Die vorliegende Erfindung betrifft Verfahren zur Herstellung von Glykoloylaniliden (Hydroxyacetaniliden) sowie neue O-Benzylglykoloylanilide und Verfahren zu deren Herstellung.

Glykoloylanilide stellen wichtige Vorprodukte zur Herstellung von Herbiziden (EP-A 300 344 und EP-A 510 479), pharmazeutischen Wirkstoffen (DE-A 083 964 und EP-A 284 388) und Fungiziden (US 4 440 780) dar.

Zu ihrer Herstellung sind bereits verschiedene Wege beschrieben worden.

Gemäß US 4 440 780 (Beispiel 3) verseift man ein Chloracetanilid mit wäßrigem Alkalihydroxyd in Gegenwart eines Lösungsmittels (Dimethoxyethan). An diese Verseifung schließt sich eine sehr aufwendige, mehrstufige Aufarbeitung an, die u.a. drei Filtrations-, zwei Extraktions- und zwei Trocknungsschritte sowie das Durchleiten von gasförmigem HCl umfaßt. Das entsprechende Glykoloylanilid wird mit einer Ausbeute von 78,1 % erhalten, eine Angabe über die Reinheit des Produktes fehlt.

Einer allgemeinen Anwendung dieses Verfahrens steht neben der unbefriedigenden Ausbeute und sehr aufwendigen Aufarbeitung eine unerwünschte Bildung von entsprechenden Ethern entgegen, die sich unter den Bedingungen dieser Verseifung aus bereits gebildetem Glykoloylanilid und noch nicht umgesetztem Chloracetanilid bilden und zu einer beträchtlichen Verringerung der Ausbeute führen. Die Bildung dieser Ether läßt sich allerdings nicht oder nur in recht geringem Umfange vermeiden.

Die DE-OS 32 22 229 beschreibt ein Verfahren zur Herstellung von mono- und disubstituierten Glykoloylamiden (Glykolsäureamiden), wobei man ein Gemisch aus Natriumchloracetat, einem tertiären Amin als Katalysator und Xylol als Verdünnungsmittel zum Sieden erhitzt, das hierbei gebildete feste Zwischenprodukt isoliert und dieses in der Struktur nicht offenbarte Festprodukt, das vermutlich ein Gemisch von Oligoglykoliden darstellt, mit einem primären oder sekundären Amin in Gegenwart eines quartären Ammoniumsalzes als Katalysator zu den entsprechenden Glykoloylamiden umsetzt.

Die Ausbeuten sind bei Einsatz (cyclo)aliphatischer Amine mäßig bis akzeptabel, im einzigen mit Zahlen belegten Beispiel, das ein Glykoloylanilid betrifft (Tabelle 1, Beispiel 6, Einsatz von N-Methylanilin), wird allerdings eine Ausbeute von lediglich 62 % angegeben.

Nachteilig an dem Verfahren ist, daß das Oligoglykolidgemisch als Festprodukt durch Filtration isoliert werden muß und daß es als chlorhaltiges Produkt in die nächste Verfahrensstufe eingesetzt wird. Zudem erweist sich die Verwendung von quartären Ammoniumsalzen als Katalysator wegen deren bioziden Eigenschaften als problematisch. Darüberhinaus ist die Ausbeute für eine Herstellung von Glykoloylaniliden unbefriedigend.

Die DE-OS 29 04 490 betrifft ein Verfahren zur Herstellung von Glykoloylamiden (α-Hydroxycarbonsäureamiden) durch Umsetzung von α-Halogencarbonsäureamiden mit Alkali- oder Erdalkali-Acetaten zu den entsprechenden α-Acetoxycarbonsäureamiden und anschließende Spaltung (Entacylierung) der α-Acetoxycarbonsäureamide. Die als Einsatzstoffe verwendeten α-Halogencarbonsäureamide erfordern allerdings eine eigene Herstellung und lassen sich beispielsweise durch Umsetzen von α-Halogencarbonsäurehalogeniden mit Ammoniak bzw. primären oder sekundären Aminen, gegebenenfalls in Gegenwart eines Säureakzeptors (Kaliumhydroxid) herstellen. Trotz der Verwendung von α-Halogencarbonsäureamiden als Einsatzmaterial handelt es sich bei dem Verfahren der DE-OS 29 04 490 um einen zweistufigen Prozeß, wobei das in der ersten Verfahrensstufe gebildete α-Acetoxycarbonsäureamid unter Abtrennung des Lösungsmittels isoliert werden muß, um anschließend in der zweiten Verfahrensstufe unter Verwendung eines aliphatischen Alkohols als Lösungsmittel in Gegenwart katalytischer Mengen eines Alkali- oder Erdalkalihydroxydes solvolytisch gespalten zu werden.

Die Ausbeuten liegen, soweit angegeben, auch beim Einsatz aromatischer Amine hoch (90 bis 99 %), die erzielte Reinheit läßt allerdings mit 97,3 bzw. 98 % (GC) noch Wünsche offen.

Nachteilig an dem Verfahren ist einerseits der Gebrauch von α-Halogencarbonsäureamiden, die in der Herstellung einen separaten Arbeitsaufwand erfordern, als Einsatzstoff und andererseits die Verwendung von mindestens zwei unterschiedlichen Lösungsmitteln, die Isolierung des α-Acetoxycarbonsäureamids und die Verwendung von quartären Ammoniumsalzen als Katalysatoren. Der infolge der in der zweiten Stufe ablaufenden Umsetzung in stöchiometrischen Mengen gebildete Essigsäureester erfordert ebenso, wie das als Katalysator verwendete biozide quartäre Ammoniumsalz sowohl in der Aufarbeitung, als auch in der Entsorgung zusätzliche Verfahrensschritte, die einen erhöhten apparativen Aufwand erfordern.

Es besteht daher ein erhebliches Interesse, ein Verfahren bereitzustellen, das die Nachteile der vorstehend beschriebenen Verfahren vermeidet und sich technisch einfach realisieren läßt. Es soll sich zudem in breitem Umfange anwenden lassen und die Herstellung von Glykoloylaniliden nicht nur in hoher Ausbeute sondern auch in hoher Reinheit ermöglichen.

Gelöst wird die Aufgabe durch ein Verfahren zur Herstellung von Glykoloylaniliden der allgemeinen Formel (G) 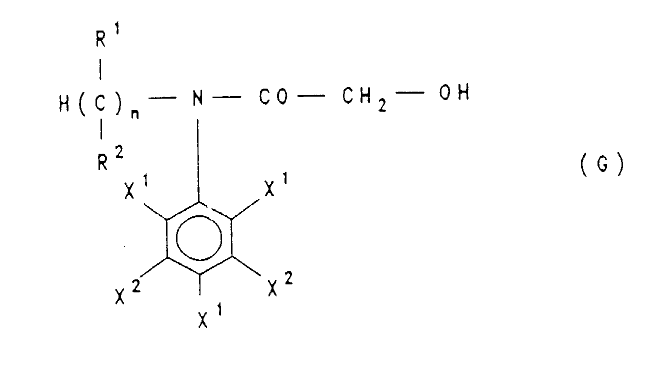 worin X¹ unabhängig für H, ein Halogen, einen Cyan-, Trifluormethyl-, Alkyl- oder Alkoxy-Rest mit jeweils 1 bis 4 Kohlenstoffatomen im Alkylrest und X² unabhängig voneinander für H, ein Halogen, einen Cyan-, Carboxyl-, Trifluormethyl-, einen gegebenenfalls substituierten Aminocarbonyl- oder Aminosulfonyl-, einen Alkyl-, Alkoxy- oder Alkoxycarbonyl-Rest mit jeweils 1 bis 4 Kohlenstoffatomen im Alkylrest, n für 0 oder 1 steht, und R¹ und R² für H, einen Alkyl-, Hydroxyalkyl-, Alkoxyalkyl- oder Acyloxyalkyl-Rest mit jeweils 1 bis 4 Kohlenstoffatomen im Alkylrest stehen.

Es ist dadurch gekennzeichnet, daß man ein Nitrobenzol der allgemeinen Formel (A) mit Wasserstoff und gegebenenfalls einer Carbonylverbindung der Formel (B) in Gegenwart eines Edelmetall enthaltenden Katalysators und eines Lösungsmittels entsprechend der Reaktionsgleichung (1) 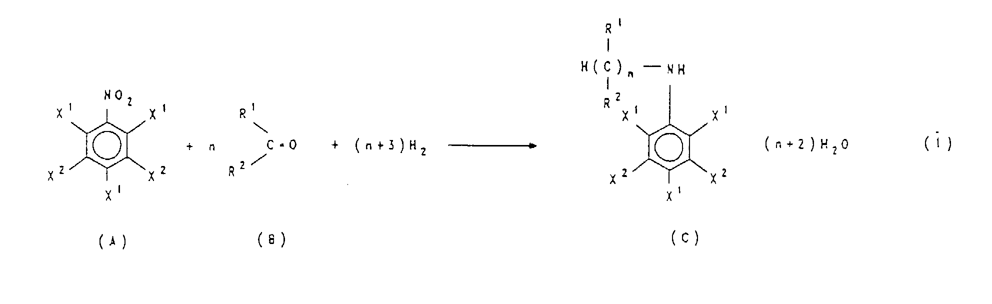 umsetzt, den Katalysator abtrennt und die Verbindung der Formel (C) mit Chloracetylchlorid entsprechend der Reaktionsgleichung (2) 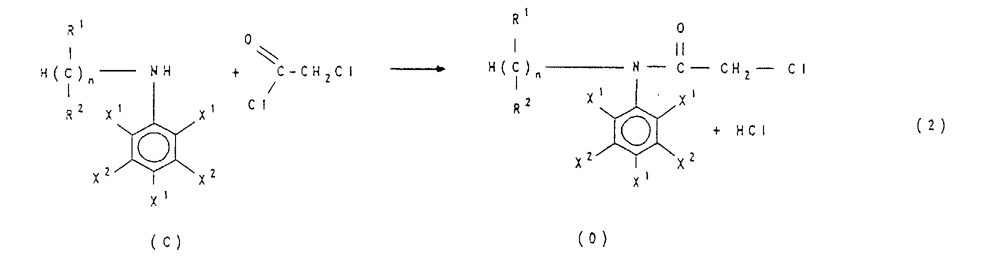 zu einer Verbindung der Formel (D) umsetzt, den gebildeten Chlorwasserstoff abtrennt, die Verbindung der Formel (D) mit einem Benzylalkohol der Formel (E), worin R³ für H, ein Halogen, einen Alkyl- oder Alkoxy-Rest mit jeweils 1 bis 4 Kohlenstoffatomen steht, und einer Base entsprechend der Reaktionsgleichung (3) 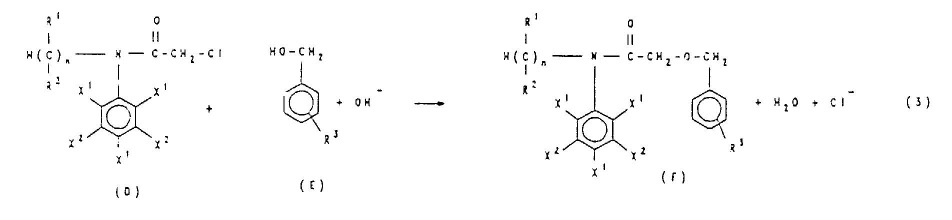 umsetzt, gegebenenfalls das aus der Base und Chlorwasserstoff gebildete Salz abtrennt, oder die Verbindung der Formel (C) mit einem O-Benzylglykoloylchlorid der Formel (K), worin R³ die vorstehend genannte Bedeutung besitzt, und gegebenenfalls mit einer Base entsprechend der Reaktionsgleichung (4) 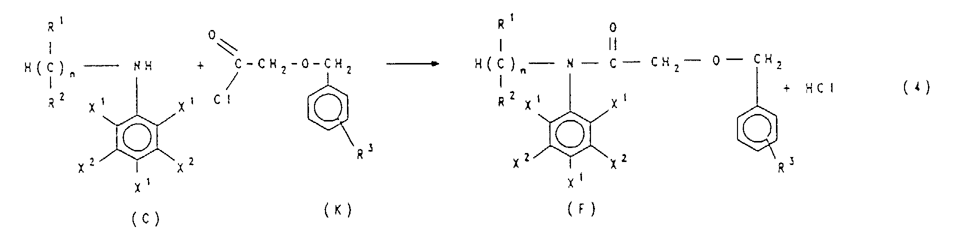 umsetzt, gegebenenfalls das aus Base und Chlorwasserstoff gebildete Salz abtrennt und das O-Benzylglykoloylanilid der Formel (F) in Gegenwart eines Edelmetall enthaltenden Katalysators mit Wasserstoff entsprechend der Reaktionsgleichung (5) 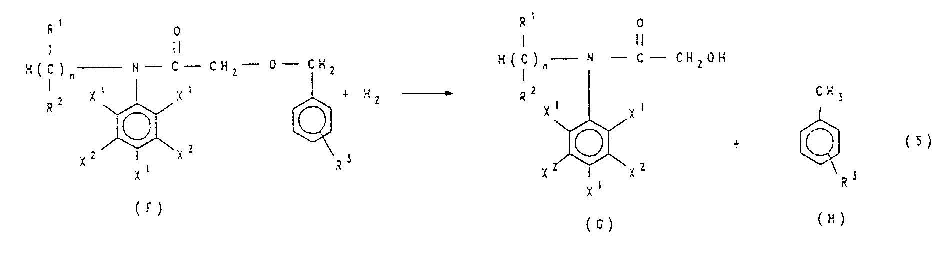 umsetzt, die gebildete Verbindung der Formel (H) abtrennt und das Glykoloylanilid der Formel (G) isoliert.

Ein Vorteil des erfindungsgemäßen Verfahrens besteht darin, daß es sich wegen der großen Zahl der als Einsatzstoff in Frage kommenden Nitrobenzole der allgemeinen Formel (A) in breitem Umfange anwenden läßt und zu einer Vielzahl entsprechender Glykoloylanilide führt. Somit ist eine hohe Flexibilität des Verfahrens gewährleistet.

Obwohl das erfindungsgemäße Verfahren über mehrere Stufen verläuft, liefert es überraschenderweise die gewünschten Glykoloylanilide nicht nur in sehr hoher Ausbeute, sondern auch in sehr hoher Reinheit. Es lassen sich ohne weiteres Ausbeuten von 90 % und darüber erzielen, wobei das Endprodukt in einer Reinheit von 98,5 % und darüber anfällt. Diese Ergebnisse sind zugleich ein Beleg dafür, daß es trotz der Empfindlichkeit der Glykoloylanilide gegenüber hydrolytischen Einflüssen überraschenderweise nicht zu einer ausbeutemindernden hydrolytischen Spaltung der Glykoloylanilide in Hydroxyessigsäure und das entsprechende Anilin kommt.

Ein weiterer Vorteil des erfindungsgemäßen Verfahrens ist, daß es nicht erforderlich ist, jedes einzelne im Verlauf der Synthese gebildete Zwischenprodukt zu isolieren und zu reinigen, um es anschließend in gereinigter Form weiterzuverarbeiten. Vielmehr ist es möglich, je nach Wunsch oder Bedarf, auf einen oder auch mehrere dieser Abtrennungsschritte und die sich daran anschließende Reinigung des jeweiligen Zwischenproduktes zu verzichten. Dies hat zur Folge, daß das erfindungsgemäße Verfahren sich in den entsprechenden Reaktionsschritten technisch besonders einfach gestaltet und sich ohne großen Aufwand durchführen läßt.

Im günstigsten Falle läßt sich das erfindungsgemäße Verfahren, ausgehend von dem entsprechenden Nitrobenzol der Formel (A), unter Verwendung eines einzigen Lösungsmittels im Sinne eines Eintopf-Verfahrens durchführen, wobei alle Reaktionsstufen ohne Isolierung und Reinigung der jeweiligen Zwischenprodukte bis zum Glykoloylanilid durchlaufen werden. Diese Verfahrensvariante stellt eine besonders vorteilhafte und technisch leicht zu realisierende Ausführungsform des erfindungsgemäßen Verfahrens dar und erfordert lediglich die Abtrennung des Edelmetall enthaltenden Katalysators und gegebenenfalls der gebildeten Salze, beispielsweise durch Filtration, und die Entfernung von gegebenenfalls heterogen vorliegendem Reaktionswasser. Wird der Edelmetall enthaltende Katalysator in Form eines Festbettes angewendet, so entfällt auch dessen Abtrennung aus dem jeweiligen Reaktionsgemisch.

Als Nitrobenzol der Formel (A) bietet sich eine Vielzahl verschiedener Nitrobenzole an. Es kommen die verschiedenen isomeren Nitroalkylbenzole, insbesondere Nitrotoluole, Nitroethylbenzole, Nitroxylole und Nitrotrimethylbenzole, die verschiedenen isomeren Nitrohalogenbenzole, Nitrodihalogenbenzole, Nitrotrihalogenbenzole und Nitrotetrahalogenbenzole, die verschiedenen isomeren Nitroanisole, Nitrophenetole, Nitrodimethoxybenzole und Nitrotrimethoxybenzole, die verschiedenen isomeren Nitrocyanbenzole und Nitrotrifluormethylbenzole, die verschiedenen isomeren Nitrobenzoesäuren, Nitrophthalsäuren, Nitroisophthalsäuren und Nitroterephthalsäuren sowie deren Alkylester und gegebenenfalls alkylsubstituierten Amide, sowie die verschiedenen isomeren, gegebenenfalls N-alkylsubstituierten, Nitrobenzolsulfonamide in Betracht.

Allgemein geeignet sind Nitrobenzole der Formel (A), worin X¹ unabhängig voneinander für H, Fluor, Chlor oder eine Trifluormethylgruppe und X² unabhängig voneinander für H, Fluor, Chlor, eine Trifluormethylgruppe oder eine gegebenenfalls substituierte Aminocarbonylgruppe steht.

Gut geeignet sind die verschiedenen isomeren Monohalogennitrobenzole und Dihalogennitrobenzole, die verschiedenen isomeren Trifluormethylnitrobenzole und die verschiedenen isomeren Nitrobenzolmonocarbonsäuren und Nitrobenzoldicarbonsäuren, sowie deren Alkylester und gegebenenfalls N-substituierten Amide.

Besonders geeignet sind die verschiedenen isomeren Monochlor- und Monofluornitrobenzole, die verschiedenen isomeren Nitrobenzotrifluoride und die verschiedenen isomeren Nitrobenzamide und Nitrobenzoldicarbonsäurediamide, insbesondere 4-Chlor- und 4-Fluornitrobenzol, 4-Nitrobenzotrifluorid und 5-Nitroisophthalsäureamide.

Man setzt das Nitrobenzol der Formel (A) in Gegenwart eines Edelmetall enthaltenden Katalysators und eines Lösungsmittels mit Wasserstoff und gegebenenfalls einer Carbonylverbindung der Formel (B) bei 0,1 bis 5, insbesondere 0,2 bis 3 MPa um. Es ist auch möglich, die Umsetzung bei höheren Drücken durchzuführen, man wird jedoch in der Regel aus Gründen einer leichteren technischen Verfahrensführung innerhalb der vorstehend genannten Druckbereiche arbeiten.

Als Carbonylverbindung der Formel (B) eignen sich aliphatische Carbonylverbindungen, insbesondere Acetaldehyd, Propionaldehyd, Aceton, Methylethylketon, Methoxyacetaldehyd und/oder Acetoxyacetaldehyd, bevorzugt Acetaldehyd und/oder Aceton.

Man setzt das Nitrobenzol der Formel (A) und die Carbonylverbindung der Formel (B) im Mol-Verhältnis 1:(1,0 bis 3,5), insbesondere 1:(1,1 bis 2,5), bevorzugt 1:(1,05 bis 1,5) ein.

Der 1 bis 10, bevorzugt 2 bis 5 Gew.-% Edelmetall enthaltende Katalysator gelangt entsprechend einer Menge von 0,01 bis 0,3, insbesondere 0,025 bis 0,15 Gew.-Teilen Edelmetall, bezogen auf 100 Teile Nitrobenzol der Formel (A), zur Anwendung.

Die entsprechend der Reaktionsgleichung (1) ablaufende Umsetzung des Nitrobenzols der Formel (A) läßt sich besonders einfach mit Hilfe eines Edelmetall enthaltenden Trägerkatalysators, der entweder in suspendierter Form oder als Festbettkatalysator Anwendung finden kann, durchführen. Geeignet als Trägermaterial sind Al₂O₃, Bimsstein, Tonerden, Kieselsäure, Kieselgur, Kieselgel, SiO₂ und/oder Aktivkohle, bevorzugt Aktivkohle.

Der das Edelmetall enthaltende Trägerkatalysator weist üblicherweise 1 bis 10, insbesondere 2 bis 5 Gew.-% Edelmetall, bezogen auf den gesamten Katalysator, auf. Es empfiehlt sich als Edelmetall enthaltenden Katalysator einen Palladium oder Platin enthaltenden Katalysator, insbesondere einen Palladium oder Platin, gegebenenfalls in sulfitierter oder sulfidierter Form, enthaltenden Trägerkatalysator, bevorzugt einen Palladium oder Platin, gegebenenfalls in sulfitierter oder sulfidierter Form, enthaltenden Aktivkohle-Katalysator zu verwenden. Die Wahl des Edelmetall enthaltenden Katalysators richtet sich in gewissem Umfange auch nach der Art des Nitrobenzols der Formel (A). Für die Umsetzung von halogenfreien Nitrobenzolen eignen sich Palladium oder Platin, insbesondere Palladium enthaltende Katalysatoren, bevorzugt entsprechende Trägerkatalysatoren, besonders bevorzugt entsprechende Katalysatoren auf Aktivkohle. Setzt man jedoch ein halogenhaltiges Nitrobenzol der Formel (A) ein, so verwendet man, um eine Halogenabspaltung sicher zu vermeiden, spezielle modifizierte Katalysatortypen, beispielsweise einen sulfitierten oder sulfidierten Platin- oder Palladiumkatalysator, insbesondere einen sulfitierten Platinkatalysator, vorzugsweise einen sulfitierten Platin-Aktivkohle-Katalysator.

Als geeignete Lösungsmittel seien aromatische Verbindungen, beispielsweise Toluol, die verschiedenen isomeren Xylole und deren Mischungen, Halogenbenzole, die verschiedenen isomeren Halogentoluole und Dihalogenbenzole sowie deren Gemische, die verschiedenen isomeren Methoxytoluole und Trimethylbenzole sowie deren Gemische genannt. Ferner lassen sich in den Hydrierstufen Alkohole, wie Methanol, Ethanol, die isomeren Propanole und Butanole sowie deren Acetate als Lösungsmittel verwenden. Gut geeignet als Lösungsmittel in allen Stufen sind Toluol, die verschiedenen isomeren Xylole und deren Gemische, die verschiedenen isomeren Halogentoluole oder Methoxytoluole und deren Gemische. Besonders einfach gestaltet sich das erfindungsgemäße Verfahren, wenn man die Verbindung (Toluolderivat) der Formel (H), die sich entsprechend der Reaktionsgleichung (5) bildet, als Lösungsmittel einsetzt.

Man setzt das Nitrobenzol der Formel (A) entsprechend der Reaktionsgleichung (1) üblicherweise bei 20 bis 100, insbesondere bei 60 bis 90°C um. Als Folge der Umsetzung entsteht die Verbindung der Formel (C). Man trennt den Katalysator und gegebenenfalls heterogen vorliegendes Reaktionswasser ab und isoliert, falls gewünscht, die Verbindung der Formel (C) beispielsweise durch Abdestillieren des Lösungsmittels, bevor man sie in die nächste Reaktionsstufe einsetzt. Nach einer besonderen Verfahrensvariante trennt man lediglich den Katalysator und das gegebenenfalls heterogen vorliegende Reaktionswasser ab und setzt das die Verbindung der Formel (C) enthaltende Reaktionsgemisch unmittelbar, ohne die Verbindung der Formel (C) zu isolieren, entsprechend der Reaktionsgleichung (2) mit Chloracetylchlorid um.

Man setzt die Verbindung der Formel (C) bei 0 bis 150, insbesondere 20 bis 100°C mit Chloracetylchlorid um. Im allgemeinen setzt man die Verbindung der Formel (C) und Chloracetylchlorid im Mol-Verhältnis 1:(1,0 bis 1,5) ein. In einer Reihe von Fällen hat es sich als nützlich erwiesen, die Verbindung der Formel (C) und Chloracetylchlorid im Molverhältnis 1:(1,05 bis 1,15) einzusetzen.

Während der Umsetzung bildet sich Chlorwasserstoff, der aus dem Reaktionsgemisch abgetrennt werden muß. Dies kann man durch Verkochen und/oder durch Zusatz einer Base, beispielsweise durch Zugabe eines Alkalihydroxids oder Alkalicarbonats, erreichen. Es hat sich besonders bewährt, den gebildeten Chlorwasserstoff durch Verkochen abzutrennen.

Nach Abschluß der Umsetzung kann man die erhaltene Verbindung der Formel (D) isolieren, ehe man sie in die nächste Reaktionsstufe einsetzt. Es ist jedoch von Vorteil, das die Verbindung der Formel (D) enthaltende Reaktionsgemisch unmittelbar, ohne die Verbindung der Formel (D) zu isolieren, entsprechend der Reaktionsgleichung (3) mit dem Benzylalkohol der Formel (E) umzusetzen.

Man verwendet als Benzylalkohol eine Verbindung der Formel (E), worin R³ für H, ein Halogen, einen Alkyl- oder Alkoxy-Rest mit jeweils 1 bis 4 Kohlenstoffatomen, insbesondere für H, Chlor, eine Methylgruppe oder Methoxygruppe steht.

Üblicherweise setzt man die Verbindung der Formel (D) und den Benzylalkohol der Formel (E) im Mol-Verhältnis 1:(1 bis 1,5) ein. In einer Reihe von Fällen hat es sich als ausreichend erwiesen, die Verbindung der Formel (D) und den Benzylalkohol der Formel (E) im Mol-Verhältnis 1:(1,02 bis 1,2) umzusetzen. Die Umsetzung der Verbindung der Formel (D) erfolgt üblicherweise bei 20 bis 200°C. In einer Reihe von Fällen genügt es, diese Umsetzung bei 20 bis 150°C durchzuführen. Man führt die Umsetzung der Verbindung der Formel (D) in Anwesenheit einer Base durch. Üblicherweise setzt man die Verbindung der Formel (D) und die Base im Mol-Verhältnis bzw. im Verhältnis ihrer Äquivalente von 1:(1,0 bis 1,5), insbesondere 1:(1,02 bis 1,2) um. Man verwendet üblicherweise ein Alkalihydroxid oder ein Alkalicarbonat als Base.

Man erhält auf diese Weise die Verbindung der Formel (F).

Alternativ läßt sich das O-Benzylglykoloylanilid der Formel (F) durch Umsetzung der Verbindung der Formel (C) mit einem O-Benzylglykoloylchlorid der Formel (K), worin R³ die vorstehend genannte Bedeutung besitzt und insbesondere für H, Chlor, eine Methylgruppe oder Methoxygruppe steht, entsprechend der Reaktionsgleichung (4), gegebenenfalls unter Zusatz einer Base, herstellen. Hierzu kann man entweder die Verbindung (C) in isolierter Form oder das die Verbindung der Formel (C) enthaltende Reaktionsgemisch unmittelbar, ohne die Verbindung der Formel (C) zu isolieren, mit dem O-Benzylglykoloylchlorid der Formel (K) umsetzen. Hierbei wird der Edelmetall enthaltende Katalysator und das gegebenenfalls heterogen anfallende Reaktionswasser zuvor abgetrennt.

Das bei dieser Verfahrensvariante eingesetzte O-Benzylglykoloylchlorid der Formel (K) läßt sich nach bekannten Methoden, beispielsweise durch Umsetzen von Chloressigsäure mit einem Alkalisalz eines Benzylalkohols und nachfolgender Chlorierung der O-Benzylglykolsäure mittels Phosphorpentachlorid (Helv. Chim. Acta 1933, 16, 1130 bis 1132) oder durch Umsetzung von Natriumchloracetat mit einem Benzylalkohol und anschließender Chlorierung der O-Benzylglykolsäure mit Thionylchlorid, Phosgen oder Phosphoroxychlorid synthetisieren.

Zur Umsetzung entsprechend der Reaktionsgleichung (4) setzt man die Verbindung der Formel (C) und das O-Benzylglykoloylchlorid der Formel (K) im Mol-Verhältnis 1:(1 bis 1,5), insbesondere 1:(1,05 bis 1,15) ein.

Im allgemeinen setzt man die Verbindung der Formel (C) mit dem Benzylglykoloylchlorid der Formel (K) bei 20 bis 150°C um. In einer Vielzahl von Fällen hat es sich bewährt, diese Reaktion bei 50 bis 130°C durchzuführen. Während der Umsetzung der Verbindung der Formel (C) mit dem O-Benzylglykoloylchlorid der Formel (K) bildet sich Chlorwasserstoff, der aus dem Reaktionsgemisch abzutrennen ist. Die Abtrennung des Chlorwasserstoffes kann durch Auskochen und/oder durch Zusatz einer Base erfolgen.

Es ist möglich, die Verbindung der Formel (C) mit dem O-Benzylglykoloylchlorid der Formel (K) unter Zusatz von einem Alkalihydroxyd und/oder Alkalicarbonat als Base umzusetzen. Auf diese Weise wandelt man den gebildeten Chlorwasserstoff bereits während der Reaktion in ein Salz um, das gegebenenfalls aus der anfallenden Reaktionsmischung, beispielsweise durch Filtration, entfernt wird.

Zur Weiterverarbeitung kann man - unabhängig von der Art der Herstellung - das O-Benzylglykoloylanilid der Formel (F) isolieren oder vorzugsweise das das O-Benzylglykoloylanilid der Formel (F) enthaltende Reaktionsgemisch unmittelbar, ohne das O-Benzylglykoloylanilid der Formel (F) zu isolieren, entsprechend der Reaktionsgleichung (5) mit Wasserstoff umsetzen. Hierfür verwendet man 1 bis 10, insbesondere 2 bis 5 Gew.-% Edelmetall enthaltenden Katalysator, in einer Menge von 0,025 bis 0,5, insbesondere 0,05 bis 0,3 Gew.-Teilen Edelmetall, bezogen auf 100 Teile O-Benzylglykoloylanilid der Formel (F). Man verwendet als Edelmetall enthaltenden Katalysator einen Palladium oder Platin, gegebenenfalls in sulfitierter Form, enthaltenden Katalysator, insbesondere einen entsprechenden Trägerkatalysator, bevorzugt einen entsprechenden Aktivkohle-Katalysator.

Die Wahl des Katalysators hängt in gewissem Umfange auch von dem umzusetzenden O-Benzylglykoloylanilid der Formel (F) ab. Bei halogenfreien O-Benzylglykoloylaniliden liefern Palladium oder Platin enthaltende Trägerkatalysatoren, insbesondere Palladium oder Platin enthaltende Aktivkohle-Katalysatoren, bevorzugt Palladium enthaltende Aktivkohle-Katalysatoren gute Ergebnisse. Beabsichtigt man halogenhaltige O-Benzylglykoloylanilide der Formel (F) umzusetzen, so empfiehlt es sich, speziell modifizierte Katalysatoren, wie sulfitierte oder sulfidierte Palladium- oder Platin-Trägerkatalysatoren, insbesondere sulfitierte Platin-Trägerkatalysatoren, bevorzugt sulfitierte Platin-Aktivkohle-Katalysatoren zu gebrauchen. Man läßt die Umsetzung bei 0,1 bis 5, insbesondere 0,2 bis 3 MPa ablaufen. Üblicherweise setzt man das O-Benzylglykoloylanilid der Formel (F) und Wasserstoff bei 20 bis 100°C um. In einer Vielzahl von Fällen hat es sich als ausreichend erwiesen, diese Umsetzung bei 30 bis 80°C vorzunehmen.

Die Edelmetall enthaltenden Katalysatoren weisen üblicherweise 1 bis 10, insbesondere 2 bis 5 Gew.-% Edelmetall, bezogen auf gesamten Katalysator, auf.

Nach der reduktiven Debenzylierung entsprechend der Reaktionsgleichung (5) wird der Edelmetall enthaltende Katalysator, beispielsweise durch Filtration, abgetrennt und die entstandene Verbindung der Formel (H) zusammen mit dem gegebenenfalls verwendeten Lösungsmittel abdestilliert. Das gewünschte Glykoloylanilid fällt als bereits sehr reines Endprodukt an, das aber durch eine Destillation oder Kristallisation zusätzlich weiter gereinigt werden kann, wobei man in der Regel nahezu analysenreine Produkte erhält.

Für die durch die Reaktionsgleichungen (2), (3) und (4) beschriebenen Acylierungs- und Benzylierungsreaktionen kommen die für die erste Reaktionsgleichung genannten Lösungsmittel in Betracht, allerdings mit der Einschränkung, daß Alkohole nicht verwendet werden können, da sie mit Säurechloriden (Chloracetylchlorid, O-Benzylglykoloylchloriden der Formel (K)) und den Chloracetaniliden der Formel (D) reagieren können und dadurch zur Bildung unerwünschter Nebenprodukte führen.

Aus dem gleichen Grund können Verbindungen der Formel (C), die in den Resten R¹ und/oder R² und/oder in den Resten X² freie Hydroxygruppen enthalten, nicht in die entsprechende Acylierungs- bzw. Benzylierungsstufe - entsprechend den Reaktionsgleichungen (2), (3) und (4) - eingesetzt werden. Diese Hydroxygruppen müssen zuvor, beispielsweise durch eine Acetylierung, geschützt werden.

Die vorliegende Erfindung betrifft ferner neue O-Benzylglykoloylanilide der Formel (F) 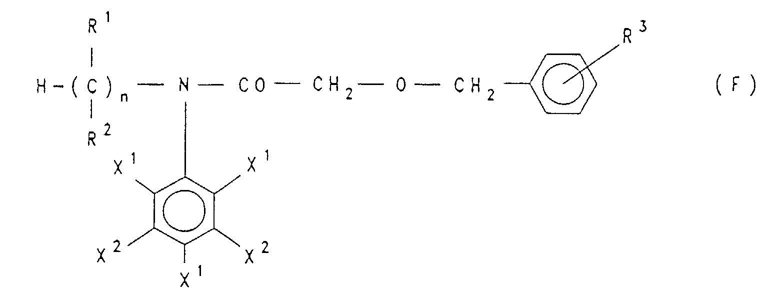 worin R¹ und R² für H, einen Alkyl-, Hydroxyalkyl-, Alkoxyalkyl oder Acyloxyalkylrest mit jeweils 1 bis 4 Kohlenstoffatomen im Alkylrest, R³ für H, ein Halogen, einen Alkyl- oder Alkoxy-Rest mit jeweils 1 bis 4 Kohlenstoffatomen im Alkylrest, X¹ unabhängig voneinander für H, ein Halogen, einen Cyan-, Trifluormethyl-, Alkyl oder Alkoxy-Rest mit jeweils 1 bis 4 Kohlenstoffatomen im Alkylrest, X² unabhängig voneinander für H, ein Halogen, einen Cyan-, Carboxyl-, Trifluormethyl-, einen gegebenenfalls substituierten Aminocarbonyl- oder Aminosulfonyl-, einen Alkyl-, Alkoxy- oder Alkoxycarbonyl-Rest mit jeweils 1 bis 4 Kohlenstoffatomen im Alkylrest und n für 0 oder 1 steht, wobei wenigstens einer der Reste X² verschieden von H ist, falls jeder der Reste X¹ eine CH₃ Gruppe darstellt, oder R³ verschieden von H ist, falls n = 0 und jeder der Reste X¹ und X² für F steht.

Es ist als überraschend anzusehen, daß mit Hilfe der neuen O-Benzylglykoloylanilide der Formel (F) die gewünschten Glykoloylanilide der Formel (G) sich mittels einer einfachen Umsetzung (Debenzylierung) bei nahezu vollständigem Umsatz und sehr hoher Selektivität entsprechend der Reaktionsgleichung (5) gewinnen lassen. Als einziges Nebenprodukt entsteht in stöchiometrischer Menge die Verbindung (Toluol oder Toluolderivat) der Formel (H), die entweder in den Prozeß zur Herstellung des Benzylalkohols der Formel (E) zurückgeführt, oder, falls das erfindungsgemäße Verfahren in der Verbindung der Formel (H) als Lösungsmittel durchgeführt wird, zur Ergänzung der bei Regeneration und Rückgewinnung auftretenden Lösungsmittelverluste verwendet werden kann. Der bei der Debenzylierung verwendete Edelmetall enthaltende Katalysator kann mehrfach in die Reaktion wieder eingesetzt werden.

Die vorliegende Erfindung betrifft ferner ein Verfahren zur Herstellung der neuen O-Benzylglykoloylanilide der vorstehend genannten Formel (F). Es ist dadurch gekennzeichnet, daß man ein Nitrobenzol der Formel (A) mit Wasserstoff und gegebenenfalls einer Carbonylverbindung der Formel (B) in Gegenwart eines Edelmetall enthaltenden Katalysators und eines Lösungsmittels entsprechend der Reaktionsgleichung (1) 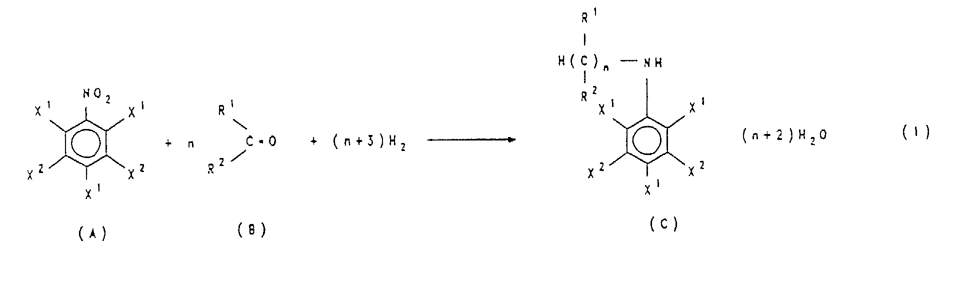 umsetzt, den Katalysator abtrennt, die Verbindung der Formel (C) mit Chloracetylchlorid entsprechend der Reaktionsgleichung (2) 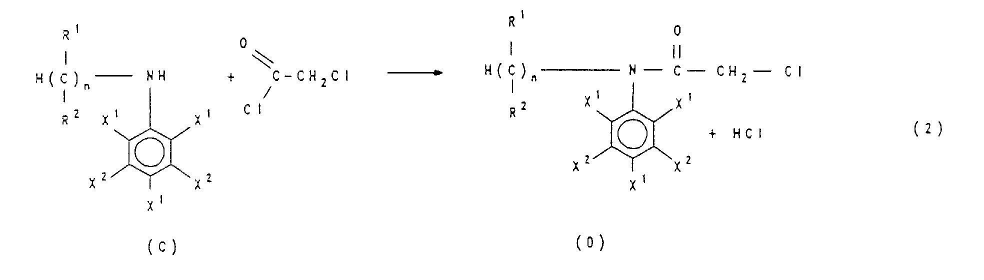 zu einer Verbindung der Formel (D) umsetzt, den gebildeten Chlorwasserstoff abtrennt, die Verbindung der Formel (D) mit einem Benzylalkohol der Formel (E), und einer Base entsprechend der Reaktionsgleichung (3) 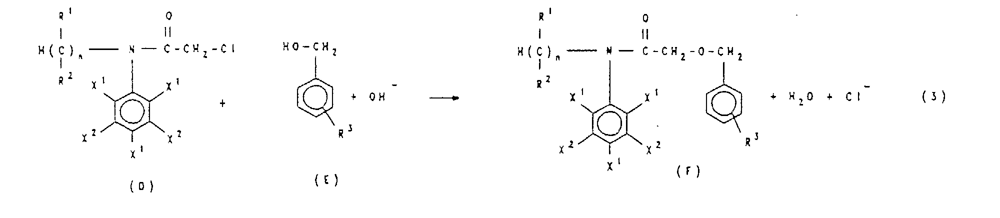 umsetzt, gegebenenfalls das aus der Base und Chlorwasserstoff gebildete Salz abtrennt, oder die Verbindung der Formel (C) mit einem O-Benzylglykoloylchlorid der Formel (K), worin R³ die vorstehend genannte Bedeutung besitzt, und gegebenenfalls mit einer Base entsprechend der Reaktionsgleichung (4) 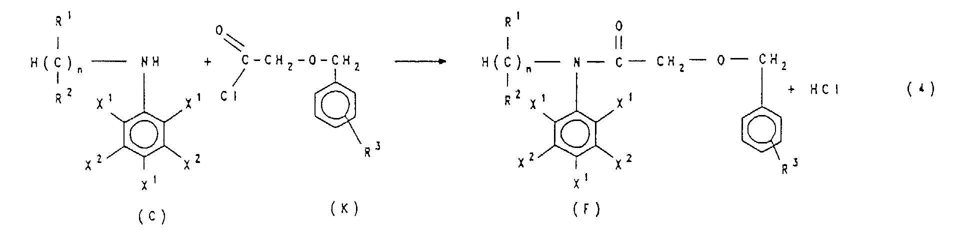 umsetzt und gegebenenfalls das aus Base und Chlorwasserstoff gebildete Salz abtrennt.

Obwohl das Verfahren - ausgehend von Nitrobenzolen der Formel (A) - über mehrere Stufen verläuft, macht es in überraschender Weise die neuen O-Benzylglykoloylanilide der Formel (F) nicht nur in sehr hoher Ausbeute, sondern auch in sehr hoher Reinheit zugänglich. Ein weiterer Vorteil dieses Verfahrens besteht darin, daß es nicht erforderlich ist, jedes einzelne im Verlauf der Synthese gebildete Zwischenprodukt zu isolieren und zu reinigen, um es anschließend in gereinigter Form weiterzuverarbeiten. Vielmehr ist es möglich, je nach Wunsch oder Bedarf, auf einen oder auch mehrere dieser Abtrennungsschritte und die sich daran anschließende Reinigung des jeweiligen Zwischenproduktes zu verzichten. Dies hat zur Folge, daß das Verfahren sich in den entsprechenden Reaktionsschritten besonders einfach gestaltet und sich ohne großen Aufwand durchführen läßt.

Im günstigsten Falle läßt sich das erfindungsgemäße Verfahren zur Herstellung der neuen O-Benzylglykoloylanilide der Formel (F) unter Verwendung eines einzigen Lösungsmittels im Sinne eines Eintopfverfahrens durchführen, wobei alle Reaktionsstufen ohne Isolierung und Reinigung der jeweiligen Zwischenprodukte durchlaufen werden. Diese Verfahrensvariante stellt eine besonders vorteilhafte und technisch leicht zu realisierende Ausführungsform des Verfahrens dar und erfordert lediglich die Abtrennung des Edelmetall enthaltenden Katalysators und gegebenenfalls der gebildeten Salze, beispielsweise durch Filtration, und die Entfernung von gegebenenfalls heterogen vorliegendem Reaktionswasser. Wird der Edelmetall enthaltende Katalysator in Form eines Festbettes angewendet, so entfällt auch dessen Abtrennung aus dem jeweiligen Reaktionsgemisch.

Das O-Benzylglykoloylanilid der Formel (F) kann in isolierter Form weiterverarbeitet werden. Es ist allerdings auch möglich, hierauf - wie zuvor bereits erwähnt - zu verzichten und das das O-Benzylglykoloylanilid enthaltende Reaktionsgemisch unmittelbar, ohne das O-Benzylglykoloylanilid der Formel (F) zu isolieren, entsprechend der Reaktionsgleichung (5) in das entsprechende Glykoloylanilid der Formel (G) umzuwandeln.

Die nachfolgenden Beispiele belegen die Erfindung, ohne sie zu beschränken.

### Experimenteller Teil

### Beispiel 1a

### Herstellung von 4-Fluor-N-isopropylanilin

In einem Hydrierautoklav werden 520 Teile Toluol, 423 Teile (3 Mol) 4-Fluornitrobenzol, 192 Teile (3,3 Mol) Aceton und 8 Teile Katalysator (5 % Pt auf Kohle, wasserfeucht, Wassergehalt 50 %) vorgelegt. Nach je dreimaligem Spülen des Gasraumes mit Stickstoff und Wasserstoff wird unter Rühren auf 80 bis 85 °C aufgeheizt und bei einem Wasserstoffdruck von 0,2 bis 1,0 MPa reduziert. Wenn nach ca. 2 Stunden die Wasserstoffaufnahme stark abfällt, wird der Druck auf 2,0 MPa erhöht und noch 30 Minuten bei 85°C nachgerührt bis kein Druckabfall mehr erfolgt. Nach Abkühlen auf 60°C wird entspannt, mit Stickstoff gespült und der Katalysator über ein Druckfilter bei 50 bis 60°C abfiltriert. Aus dem Filtrat wird die wäßrige Phase abgetrennt und so viel Toluol abdestilliert, bis der Wassergehalt die Lösung < 0,02 % beträgt. Die Lösung wird entsprechend Beispiel 2 weiterverarbeitet.

Alternativ kann die Lösung destillativ aufgearbeitet und das Produkt isoliert werden. Ausbeute 97-98 %; Reingehalt nach GC.: ≥ 99,2 %; Siedepunkt: 91 bis 92°C/10 Torr.

### Beispiel 1b

### Herstellung von 4-Fluor-N-isopropylanilin

Entsprechend Beispiel 1a werden an Stelle von 4-Fluor-nitrobenzol 333 Teile (3 Mol) 4-Fluoranilin vorgelegt und bei 80 bis 85°C und 1,0 bis 2,0 MPa Wasserstoffdruck reduziert.

Die Aufarbeitung des Reaktionsgemisches und die Weiterverarbeitung werden wie in Beispiel 1a angegeben, durchgeführt.

### Beispiel 2

### Herstellung von 4-Fluor-N-isopropyl-N-chloracetylanilin

Zu 920 Teilen 4-Fluor-N-isopropylanilin-Lösung (∼ 3 Mol 4-Fluor-N-isopropylanilin) aus Beispiel 1 werden innerhalb von 2 Stunden bei 10 bis 20°C 357,5 Teile (3,2 Mol) Chloracetylchlorid zugetropft. Es wird eine Stunde bei 20°C nachgerührt und anschließend wird durch Aufheizen auf ca. 90°C der Hauptanteil des entstandenen Chlorwasserstoff ausgegast.

Anschließend wird bei Raumtemperatur mit Natronlauge ein pH-Wert von 7 eingestellt, von ausgefallenem Natriumchlorid abfiltriert und die Wasserphase abgetrennt.

Die Lösung (1110 Teile) wird entsprechend Beispiel 3 weiterverarbeitet.

Alternativ kann durch einfache Vakuumdestillation das Reaktionsprodukt isoliert werden. Ausbeute: 94 %; Reingehalt nach GC: 98,5 %; Siedepunkt: 113-114°C/1 bis 2 Torr.

### Beispiel 3a

### Herstellung von 4-Fluor-N-isopropyl-N-benzyloxyacetylanilin

Zu ca. 1110 Teilen 4-Fluor-N-isopropyl-N-chloracetylanilin-Lösung aus Beispiel 2 mit einem Gehalt von 3 Mol 4-Fluor-N-isopropyl-N-chloracetylanilin und 389 Teilen (3,6 Mol) Benzylalkohol werden in einem Rührapparat innerhalb von zwei Stunden 144 Teile (3,6 Mol) Ätznatron kontinuierlich oder portionsweise bei 25 bis 30°C unter Kühlung zugegeben. Danach wird auf 90°C aufgeheizt und drei Stunden bei dieser Temperatur nachgerührt.

Zur Aufarbeitung wird das Reaktionsgemisch auf 20 bis 50°C abgekühlt, mit Salzsäure neutralisiert (pH 6 bis 7), vom Salz abgetrennt und das Salz dreimal mit je 60 Teilen Toluol gewaschen. Waschtoluol und Filtrat werden vereinigt. Wasser, Lösungsmittel und überschüssiger Benzylalkohol werden abdestilliert und das Sumpfprodukt anschließend zur Herstellung von 4-Fluor-N-isopropyl-N-hydroxyacetyl-anilin weiterverarbeitet.

Alternativ kann das Benzyloxy-Derivat durch Vakuumdestillation isoliert werden. Ausbeute, bezogen auf eingesetztes 4-Fluor-nitrobenzol 86 % der Theorie; Erstarrungspunkt: 87,8°C; Siedepunkt: 200°C/2 bis 3 Torr; Reingehalt nach GC: ≥ 97 %.

### Vergleichsbeispiel 3b

Die DE-OS 29 04 490 beschreibt die Umsetzung von Chloracetaniliden mit wasserfreiem Natriumacetat zu Acetoxyacetaniliden und deren Verseifung zu Glykoloylaniliden.

Überträgt man dieses Verfahren auf die Herstellung von Acetoxyacetyl-N-isopropyl-4-fluoranilid und setzt als Edukt 229,5 g (1 Mol) Chloressigsäure-N-isopropyl-4-fluoranilid mit 82 g (1 Mol) wasserfreiem Natriumacetat in 320 ml Toluol bei 115 bis 120°C um, so beträgt der Umsatz nach einer Reaktionszeit von 10 Stunden max. 10 %. Selbst bei einem Einsatzverhältnis Edukt zu Natriumacetat von 1:3 wird keine wesentliche Reaktionsbeschleunigung beobachtet.

Vergleiche nachfolgenden Tabellen I und II

**Tabelle I**

| Einsatzverhältnis Edukt zu Natriumacetat 1:1 | | |
|---|---|---|
| Reaktionszeit in Stunden | % Edukt* | % Produkt* |
| 8 | 93 | 4,5 |
| 16 | 87,5 | 9,5 |
| 24 | 84 | 14 |
| 40 | 71 | 28 |
| 75 | 42 | 57,5 |
| 105 | 24 | 75 |
| 150 | 11,5 | 87,5 |
| 175 | 1,7 | 98,0 |
| 180 | 0,5 | 98,9 |

| | | |
|---|---|---|
| * %-Werte nach GC-Analyse ohne inneren Standard | | |

**Tabelle II**

| Einsatzverhältnis Edukt zu Natriumacetat 1:3 | | |
|---|---|---|
| Reaktionszeit in Stunden | % Edukt* | % Produkt* |
| 5,5 | 92,2 | 7,0 |
| 11 | 84,5 | 14,8 |
| 20 | 75 | 24,4 |
| 35 | 54 | 45,5 |
| 50 | 32,8 | 66,5 |
| 75 | 8,3 | 91,2 |
| 95 | 0,4 | 98,9 |

| | | |
|---|---|---|
| * %-Werte nach GC-Analyse ohne inneren Standard | | |

### Beispiel 4a

### Herstellung von N-Isopropyl-N-(4-fluorphenyl)-glykolsäureamid (4-Fluor-N-isopropyl-N-hydroxyacetyl-anilin)

In einem Hydrierautoklav werden 301 Teile (1 Mol) 4-Fluor-N-isopropyl-N-benzyloxyacetyl-anilin als Sumpfprodukt aus Beispiel 3, 600 Teile Methanol und 12 Teile Palladium-Katalysator (5 % Pd auf Kohle, wasserfeucht, Wassergehalt 50 % ) vorgelegt. Nach Spülen des Gasraumes mit Stickstoff und anschließend mit Wasserstoff wird unter Rühren auf 70 bis 100°C aufgeheizt und bei einem Wasserstoffdruck von 0,5 bis 2,0 MPa reduziert. Nach Ende der Wasserstoffaufnahme läßt man ca. 30 Minuten bei 100°C und 2,0 MPa Wasserstoffdruck nachreagieren. Anschließend wird auf 30 bis 50°C abgekühlt, entspannt, mit Stickstoff gespült und der Pd/C-Katalysator über ein Druckfilter abgetrennt. Aus dem Filtrat wird das Lösungsmittel abdestilliert und das Produkt durch Vakuumdestillation isoliert.

Ausbeute: 191 Teile entsprechend 90,5 % der Theorie, bezogen auf eingesetztes Benzyloxy-Derivat. Siedepunkt: 125 bis 126°C bei 2 bis 3 Torr; Erstarrungspunkt: 59,5°C; Reingehalt nach GC: ≥ 98 %.

### Beispiel 4b

Anstelle von Methanol können auch andere Lösungsmittel wie Toluol oder Xylol eingesetzt und die Hydrogenolyse wie unter 4a beschrieben, durchgeführt werden.

### Beispiele 5 bis 8

### Herstellung von O-Benzylglykoloylaniliden der allgemeinen Formel (F)

### Beispiel 5

### Herstellung von O-Benzylglykolsäure-4-fluoranilid

In einem 250 ml-Kolben wird bei Raumtemperatur zu einer Lösung von 2,22 g (0,02 mol) 4-Fluoranilin in 80 ml Toluol eine Lösung von 4,06 g (0,022 mol) O-Benzylglykolsäurechlorid in 20 ml Toluol getropft. Es wird 5 h bei 50 °C gerührt. Anschließend wird der Kolbeninhalt unter reduziertem Druck zur Trockene eingedampft und in Acetonitril aufgenommen. Die Acetonitrilphase wird filtriert und das Filtrat im Vakuum zur Trockene eingedampft. Man erhält 3 g (58 % d.Th.) O-Benzylglykolsäure-4-fluoranilid als gelbes Öl mit einem Reingehalt von 97,8 % (HPLC). n_{D}²¹ = 1,5573.

### O-Benzylglykolsäure-4-fluoranilid

¹H-NMR (300 MHz, CDCl₃) δ 4.1 (s,2H), 4.6 (s,2H), 7.0 (m,2H), 7.3-7.45 (m,5H), 7.5 (m,2H), 8.3 (s,br,1H); ¹³C-NMR (75 MHz, CDCl₃) 167.69, 161.20, 136.53, 133.10, 128.78, 128.28, 128.10, 121.66, 115.54, 73.90, 69.61; ¹⁹F-NMR (282 MHz, CDCl₃)-118.16 m; IR (kapillar) 3380, 3060, 3030, 2910, 2960, 1735, 1680, 1610, 1535, 1510, 1410, 1210, 1115, 835, 795, 740, 700 cm⁻¹; MS (70 eV) m/z 260 ([M+H]⁺), 153, 107, 91 (100 %).

### Beispiel 6

### Herstellung von O-Benzylglykolsäure-2-methoxyanilid

In einem 250 ml-Kolben werden 2,46 g (0,02 mol) o-Anisidin in 80 ml Toluol vorgelegt. Bei Raumtemperatur wird eine Lösung von 4,06 g (0,022 mol) O-Benzylglykolsäurechlorid in 20 ml Toluol zugetropft. Anschließend wird die Reaktionsmischung 1 h auf Rückflußtemperatur gehalten. Das Toluol wird im Vakuum abgezogen und der Rückstand destilliert (bis 190 °C bei 2 Torr). Man erhält 3 g (55 % d. Th.) O-Benzylglykolsäure-2-methoxyanilid als violettes Öl mit einem Reingehalt von 97,0 % (GC). n_{D}²¹ = 1,5762.

### O-Benzylglykolsäure-2-methoxyanilid

¹H-NMR (300 MHz, CDCl₃) δ 3.85 (s,3H), 4.1 (s,2H), 4.65 (s,2H), 6.83-7.05 (m,3H), 7.24-7.44 (m,5H), 8.4 (d, 1H, ³J=8.0 Hz, ⁴J=1.9 Hz), 9.05 (s,br,1H); ¹³C-NMR (75 MHz, CDCl₃) 167.54, 148.26, 136.97, 128.60, 128.20, 127.77, 127.07, 124.05, 121.11, 119.82, 110.10, 73.68, 70.07, 55.72; IR (kapillar) 3390, 3065, 3030, 2940, 2900, 2840, 1690, 1600, 1530, 1460, 1250, 1115, 750, 700 cm⁻¹; MS (70 eV) m/z 271 (M⁺), 165, 123, 108, 91 (100%).

### Beispiel 7

### Herstellung von O-Benzylglykolsäure-4-methoxyanilid

In einem 250 ml-Kolben werden 2,46 g (0,02 mol) p-Anisidin in 80 ml Toluol vorgelegt. Bei Raumtemperatur wird eine Lösung von 4,06 g (0,022 mol) O-Benzylglykolsäurechlorid in 20 ml Toluol zugetropft. Die Reaktionsmischung wird anschließend hochgeheizt und 1 h lang unter Rückfluß gekocht. Das Toluol wird im Vakuum abgezogen und der Rückstand bei 200 °C und 2 Torr destilliert. Man erhält 3 g (55 % d.Th.) O-Benzylglykolsäure-4-methoxyanilid als blaßviolette Kristalle mit einem Schmelzpunkt von 68,5 °C und einem Reingehalt von 100 96 (GC).

### O-Benzylglykolsäure-4-methoxyanilid

¹H-NMR (300 MHz, DMSO) δ 3.7 (s,3H), 4.1 (s,2H), 4.6 (s,2H), 6.9 (m,2H), 7.25-7.45 (m,5H), 7.55 (m,2H), 9.6 (s,1H); ¹³C-NMR (75 MHz, DMSO) 167.53, 155.59, 137.84, 131.63, 128.39, 127.92, 127.76, 121.50, 113.88, 72.53, 69.60, 55.28; IR (KBr) 3300, 3010, 2950, 2830, 1665, 1595, 1520, 1240, 1100, 820 cm⁻¹; MS (70 eV) m/z 271 (M⁺, 100 %), 165, 150, 121, 91.

### Beispiel 8

### Herstellung von O-Benzylglykolsäure-3,5-dimethylanilid

In einem 250 ml-Kolben werden 1,48 g (0,012 mol) 3,5-Dimethylanilin in 80 ml Toluol vorgelegt. Bei 50 °C wird eine Lösung von 2,50 g (0,014 mol) O-Benzylgykolsäurechlorid in 20 ml Toluol zugetropft. Anschließend wird 3 h auf Rückflußtemperatur erhitzt und die auf Raumtemperatur abgekühlte Reaktionsmischung mit gesättigter Natriumhydrogencarbonat-Lösung und Wasser ausgeschüttelt. Die organische Phase wird über Natriumsulfat getrocknet und das Toluol im Vakuum abgezogen. Weitere flüchtige Bestandteile werden in einer Kugelrohrdestillation (bis 250 °C, 3 Torr) im Vakuum entfernt. Als Destillationsrückstand fallen
2,15 g (67 % d.Th.) O-Benzylglykolsäure-3,5-dimethylanilid als orangefarbenes Öl mit einem Reingehalt von 94,3 % (GC) an. n_{D}²¹ = 1,5768.

### O-Benzylglykolsäure-3,5-dimethylanilid

¹H-NMR (300 MHz, CDCl₃) δ 2.3 (s,6H), 4.1 (s,2H), 4.6 (s,2H), 6.8 (m,1H), 7.2 (s,2H), 7.3-7.5 (m,5H), 8.2 (s,br,1H); ¹³C-NMR (75 MHz, CDCl₃) δ 167.37, 138.74, 136.97, 136.68, 128.74, 128.39, 128.04, 126.28, 117.61, 73.78, 69.76, 21.34; IR (kapillar) 3400, 3300, 3030, 2920, 2860, 1690, 1615, 1545, 1100, 840, 740, 700 cm⁻¹; MS (70 eV) m/z 269 (M⁺), 210, 163 (100 %), 134, 121, 105, 91, 77.

## Patentansprüche

1. Verfahren zur Herstellung von Glykoloylaniliden der allgemeinen Formel (G) 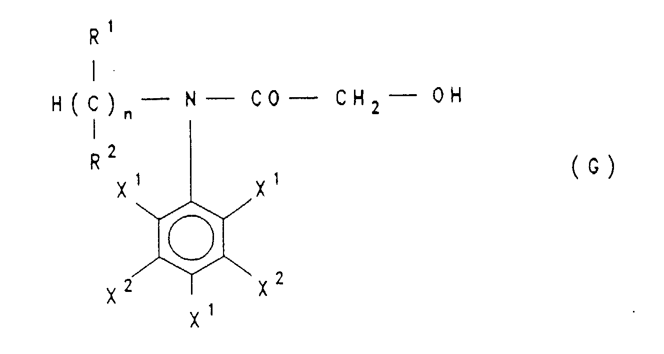 worin X¹ unabhängig für H, ein Halogen, einen Cyan-, Trifluormethyl-, Alkyl- oder Alkoxy-Rest mit jeweils 1 bis 4 Kohlenstoffatomen im Alkylrest und X² unabhängig voneinander für H, ein Halogen, einen Cyan-, Carboxyl-, Trifluormethyl-, einen gegebenenfalls substituierten Aminocarbonyl- oder Aminosulfonyl-, einen Alkyl-, Alkoxy- oder Alkoxycarbonyl-Rest mit jeweils 1 bis 4 Kohlenstoffatomen im Alkylrest, n für 0 oder 1 steht, und R¹ und R² für H, einen Alkyl-, Hydroxyalkyl-, Alkoxyalkyl- oder Acyloxyalkyl-Rest mit jeweils 1 bis 4 Kohlenstoffatomen im Alkylrest stehen, dadurch gekennzeichnet, daß man ein Nitrobenzol der allgemeinen Formel (A) mit Wasserstoff und gegebenenfalls einer Carbonylverbindung der Formel (B) in Gegenwart eines Edelmetall enthaltenden Katalysators und eines Lösungsmittels entsprechend der Reaktionsgleichung (1) 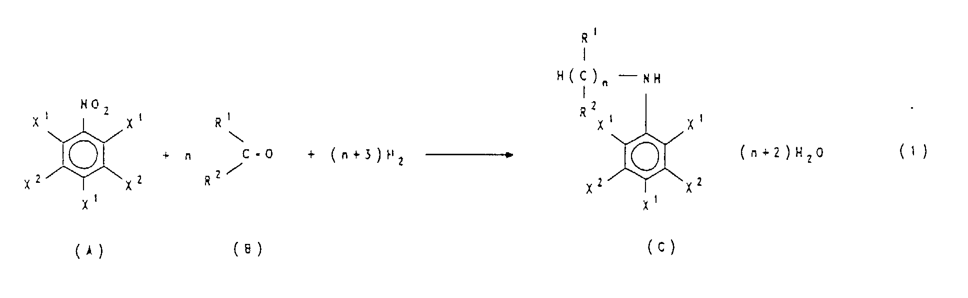 umsetzt, den Katalysator abtrennt und die Verbindung der Formel (C) mit Chloracetylchlorid entsprechend der Reaktionsgleichung (2) 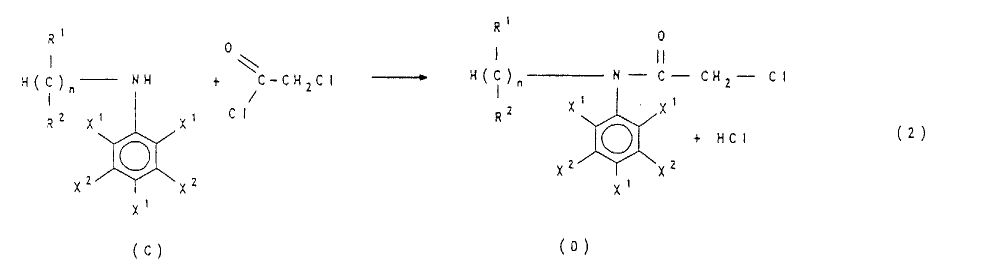 zu einer Verbindung der Formel (D) umsetzt, den gebildeten Chlorwasserstoff abtrennt, die Verbindung der Formel (D) mit einem Benzylalkohol der Formel (E), worin R³ für H, ein Halogen, einen Alkyl- oder Alkoxy-Rest mit jeweils 1 bis 4 Kohlenstoffatomen steht, und einer Base entsprechend der Reaktionsgleichung (3) 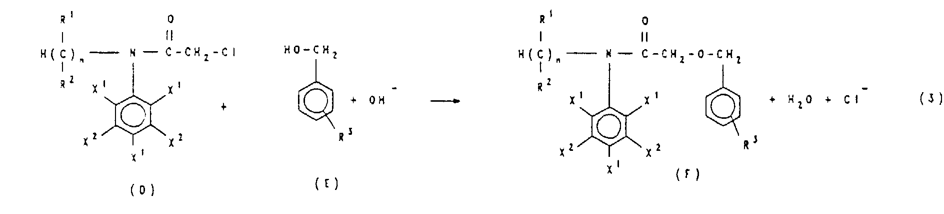 umsetzt, gegebenenfalls das aus der Base und Chlorwasserstoff gebildete Salz abtrennt, oder die Verbindung der Formel (C) mit einem O-Benzylglykoloylchlorid der Formel (K), worin R³ die vorstehend genannte Bedeutung besitzt, und gegebenenfalls mit einer Base entsprechend der Reaktionsgleichung (4) 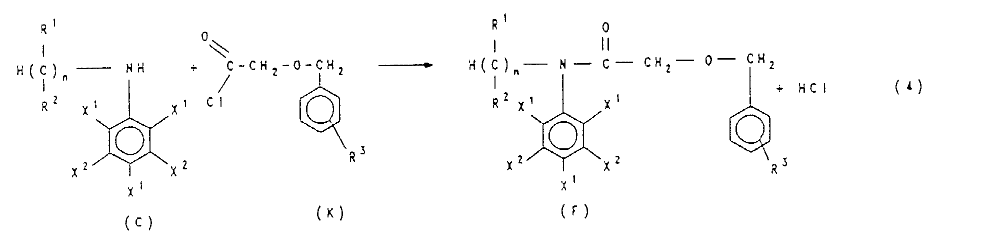 umsetzt, gegebenenfalls das aus Base und Chlorwasserstoff gebildete Salz abtrennt und das O-Benzylglykoloylanilid der Formel (F) in Gegenwart eines Edelmetall enthaltenden Katalysators mit Wasserstoff entsprechend der Reaktionsgleichung (5) 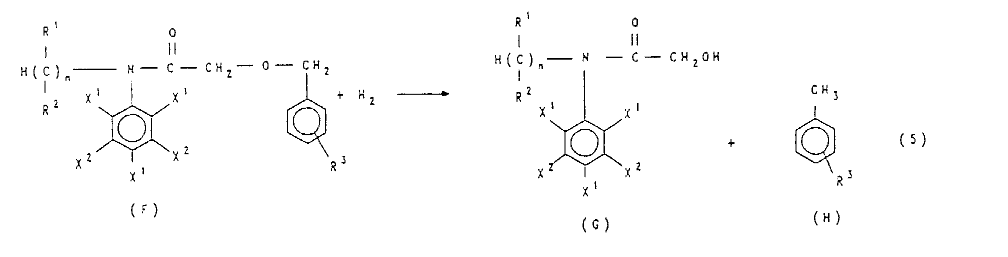 umsetzt, die gebildete Verbindung der Formel (H) abtrennt und das Glykoloylanilid der Formel (G) isoliert.

2. Verfahren nach Anspruch 1, dadurch gekennzeichnet, daß man ein Nitrobenzol der Formel (A), worin X¹ unabhängig voneinander für H, Fluor, Chlor oder eine Trifluormethylgruppe und X² unabhängig voneinander für H, Fluor, Chlor, eine Trifluormethyl- oder eine gegebenenfalls substituierte Aminocarbonylgruppe steht, einsetzt.

3. Verfahren nach Anspruch 1 oder 2, dadurch gekennzeichnet, daß man das Nitrobenzol der Formel (A) mit Wasserstoff bei 0,1 bis 5, insbesondere 0,2 bis 3 MPa umsetzt.

4. Verfahren nach einem oder mehreren der Ansprüche 1 bis 3, dadurch gekennzeichnet, daß man das Nitrobenzol der Formel (A) und die Carbonylverbindung der Formel (B) im Mol-Verhältnis 1:(1,0 bis 3,5), insbesondere 1:(1,1 bis 2,5), bevorzugt 1:(1,05 bis 1,5) einsetzt.

5. Verfahren nach einem oder mehreren der Ansprüche 1 bis 4, dadurch gekennzeichnet, daß man den 1 bis 10, insbesondere 2 bis 5 Gew.-% Edelmetall enthaltenden Katalysator in einer Menge von 0,01 bis 0,3, insbesondere 0,025 bis 0,15 Gew.-Teilen Edelmetall, bezogen auf 100 Teile Nitrobenzol der Formel (A), einsetzt.

6. Verfahren nach einem oder mehreren der Ansprüche 1 bis 5, dadurch gekennzeichnet, daß man als Edelmetall enthaltenden Katalysator einen Palladium oder Platin, gegebenenfalls in sulfitierter oder sulfidierter Form, enthaltenden Trägerkatalysator, insbesondere einen Palladium- oder Platin, gegebenenfalls in sulfitierter oder sulfidierter Form, enthaltenden Aktivkohle-Katalysator einsetzt.

7. Verfahren nach einem oder mehreren der Ansprüche 1 bis 6, dadurch gekennzeichnet, daß man als Lösungsmittel Toluol, die verschiedenen isomeren Xylole, deren Gemische, die verschiedenen isomeren Halogentoluole, deren Gemische oder die Verbindung der Formel (H) einsetzt.

8. Verfahren nach einem oder mehreren der Ansprüche 1 bis 7, dadurch gekennzeichnet, daß man das Nitrobenzol bei 20 bis 100, insbesondere 60 bis 90 °C umsetzt.

9. Verfahren nach einem oder mehreren der Ansprüche 1 bis 8, dadurch gekennzeichnet, daß man das die Verbindung der Formel (C) enthaltende Reaktionsgemisch unmittelbar, ohne die Verbindung der Formel (C) zu isolieren, mit Chloracetylchlorid umsetzt.

10. Verfahren nach einem oder mehreren der Ansprüche 1 bis 9, dadurch gekennzeichnet, daß man die Verbindung der Formel (C) bei 0 bis 150, insbesondere 20 bis 100 °C mit Chloracetylchlorid umsetzt.

11. Verfahren nach einem oder mehreren der Ansprüche 1 bis 10, dadurch gekennzeichnet, daß man die Verbindung der Formel (C) und Chloracetylchlorid im Mol-Verhältnis 1: (1,0 bis 1,5), insbesondere 1:(1,05 bis 1,15) umsetzt.

12. Verfahren nach einem oder mehreren der Ansprüche 1 bis 11, dadurch gekennzeichnet, daß man den gebildeten Chlorwasserstoff durch Verkochen und/oder durch Zusatz einer Base, abtrennt.

13. Verfahren nach einem oder mehreren der Ansprüche 1 bis 12, dadurch gekennzeichnet, daß man den gebildeten Chlorwasserstoff durch Verkochen abtrennt.

14. Verfahren nach einem oder mehreren der Ansprüche 1 bis 13, dadurch gekennzeichnet, daß man das die Verbindung der Formel (D) enthaltende Reaktionsgemisch unmittelbar, ohne die Verbindung der Formel (D) zu isolieren, mit dem Benzylalkohol der Formel (E) umsetzt.

15. Verfahren nach einem oder mehreren der Ansprüche 1 bis 14, dadurch gekennzeichnet, daß man als Benzylalkohol eine Verbindung der Formel (E), worin R³ für H, Chlor, eine Methyl- oder Methoxygruppe steht, einsetzt.

16. Verfahren nach einem oder mehreren der Ansprüche 1 bis 15, dadurch gekennzeichnet, daß man die Verbindung der Formel (D) und den Benzylalkohol der Formel (E) im Mol-Verhältnis 1:(1 bis 1,5), insbesondere 1:(1,02 bis 1,2) einsetzt.

17. Verfahren nach einem oder mehreren der Ansprüche 1 bis 16, dadurch gekennzeichnet, daß man die Verbindung der Formel (D) bei 20 bis 200, insbesondere 20 bis 150 °C umsetzt.

18. Verfahren nach einem oder mehreren der Ansprüche 1 bis 17, dadurch gekennzeichnet, daß man die Verbindung der Formel (D) und die Base im Molverhältnis 1:(1,0 bis 1,5), insbesondere 1:(1,02 bis 1,2) mit dem Benzylalkohol der Formel (E) umsetzt.

19. Verfahren nach einem oder mehreren der Ansprüche 1 bis 18, dadurch gekennzeichnet, daß man ein Alkalihydroxid oder ein Alkalicarbonat als Base einsetzt.

20. Verfahren nach einem oder mehreren der Ansprüche 1 bis 8, dadurch gekennzeichnet, daß man das die Verbindung (C) enthaltende Reaktionsgemisch umittelbar, ohne die Verbindung der Formel (C) zu isolieren, mit einem O-Benzylglykoloylchlorid der Formel (K) umsetzt.

21. Verfahren nach einem oder mehreren der Ansprüche 1 bis 8 und 20, dadurch gekennzeichnet, daß man ein O-Benzylglykoloylchlorid der Formel (K), worin für R³ H, Chlor, eine Methyl- oder eine Methoxygruppe steht, einsetzt.

22. Verfahren nach einem oder mehreren der Ansprüche 1 bis 8 und 20 bis 21, dadurch gekennzeichnet, daß man die Verbindung der Formel (C) und das O-Benzylglykoloylchlorid der Formel (K) im Molverhältnis 1:(1 bis 1,15), insbesondere 1:(1,05 bis 1,15) umsetzt.

23. Verfahren nach einem oder mehreren der Ansprüche 1 bis 8 und 20 bis 22, dadurch gekennzeichnet, daß man die Verbindung der Formel (C) mit dem O-Benzylglykoloylchlorid der Formel (K) bei 20 bis 150, insbesondere 50 bis 130 °C umsetzt.

24. Verfahren nach einem oder mehreren der Ansprüche 1 bis 8 und 20 bis 23, dadurch gekennzeichnet, daß man den bei Umsetzung der Verbindung der Formel (C) mit dem O-Benzylglykoloylchlorid der Formel (K) gebildeten Chlorwasserstoff durch Auskochen und/oder durch Zusatz einer Base abtrennt.

25. Verfahren nach einem oder mehreren der Ansprüche 1 bis 8 und 20 bis 24, dadurch gekennzeichnet, daß man die Verbindung der Formel (C) mit dem O-Benzylglykoloylchlorid der Formel (K) unter Zusatz von einem Alkalihydroxid und/oder Alkalicarbonat als Base umsetzt.

26. Verfahren nach einem oder mehreren der Ansprüche 1 bis 25, dadurch gekennzeichnet, daß man das das O-Benzylglykoloylanilid der Formel (F) enthaltende Reaktionsgemisch unmittelbar, ohne das O-Benzylglykoloylanilid der Formel (F) zu isolieren, mit Wasserstoff umsetzt.

27. Verfahren nach einem oder mehreren der Ansprüche 1 bis 26, dadurch gekennzeichnet, daß man 1 bis 10, insbesondere 2 bis 5 Gew.-% Edelmetall enthaltenden Katalysator, in einer Menge von 0,025 bis 0,5, insbesondere 0,05 bis 0,3 Gew.-Teilen Edelmetall, bezogen auf 100 Teile O-Benzylglykoloylanilid der Formel (F), einsetzt.

28. Verfahren nach einem oder mehreren der Ansprüche 1 bis 27, dadurch gekennzeichnet, daß man das O-Benzylglykoloylanilid der Formel (F) in Gegenwart eines Palladium oder Platin, gegebenenfalls in sulfitierter Form, enthaltenden Trägerkatalysators als Edelmetall enthaltenden Katalysator mit Wasserstoff umsetzt.

29. Verfahren nach einem oder mehreren der Ansprüche 1 bis 28, dadurch gekennzeichnet, daß man das O-Benzylglykoloylanilid der Formel (F) bei 0,1 bis 5, insbesondere 0,2 bis 3 MPa umsetzt.

30. Verfahren nach einem oder mehreren der Ansprüche 1 bis 29, dadurch gekennzeichnet, daß man das O-Benzylglykoloylanilid der Formel (F) bei 20 bis 100, insbesondere 30 bis 80 °C umsetzt.

31. O-Benzylglykoloylanilide der allgemeinen Formel (F) 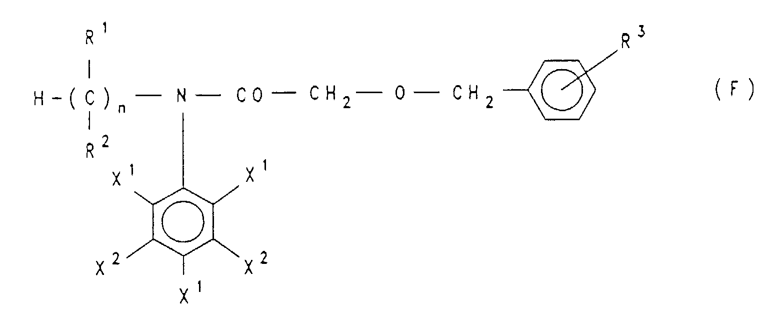 worin R¹ und R² für H, einen Alkyl-, Hydroxyalkyl-, Alkoxyalkyl oder Acyloxyalkylrest mit jeweils 1 bis 4 Kohlenstoffatomen im Alkylrest, R³ für H, ein Halogen, einen Alkyl- oder Alkoxy-Rest mit jeweils 1 bis 4 Kohlenstoffatomen im Alkylrest, X¹ unabhängig voneinander für H, ein Halogen, einen Cyan-, Trifluormethyl-, Alkyl oder Alkoxy-Rest mit jeweils 1 bis 4 Kohlenstoffatomen im Alkylrest, X² unabhängig voneinander für H, ein Halogen, einen Cyan-, Carboxyl-, Trifluormethyl-, einen gegebenenfalls substituierten Aminocarbonyl- oder Aminosulfonyl-, einen Alkyl-, Alkoxy- oder Alkoxycarbonyl-Rest mit jeweils 1 bis 4 Kohlenstoffatomen im Alkylrest und n für 0 oder 1 steht, wobei wenigstens einer der Reste X² verschieden von H ist, falls jeder der Reste X¹ eine CH₃ Gruppe darstellt, oder R³ verschieden von H ist, falls n = 0 und jeder der Reste X¹ und X² für F steht.

32. Verfahren zur Herstellung der O-Benzylglykoloylanilide der Formel (F), wie in Anspruch 31 angegeben, dadurch gekennzeichnet, daß man ein Nitrobenzol der Formel (A) mit Wasserstoff und gegebenenfalls einer Carbonylverbindung der Formel (B) in Gegenwart eines Edelmetall enthaltenden Katalysators und eines Lösungsmittels entsprechend der Reaktionsgleichung (1) 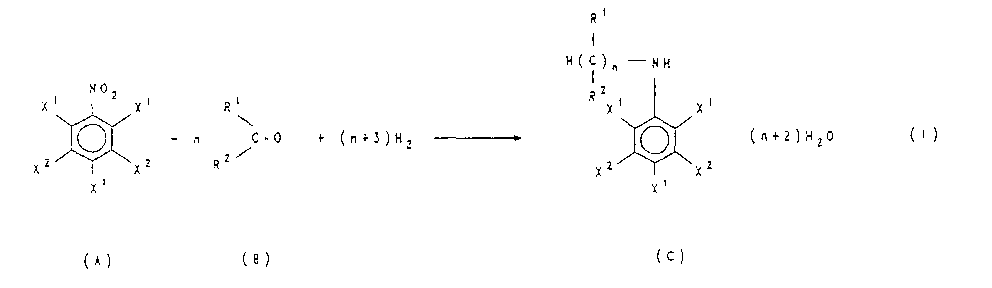 umsetzt, den Katalysator abtrennt, die Verbindung der Formel (C) mit Chloracetylchlorid entsprechend der Reaktionsgleichung (2) 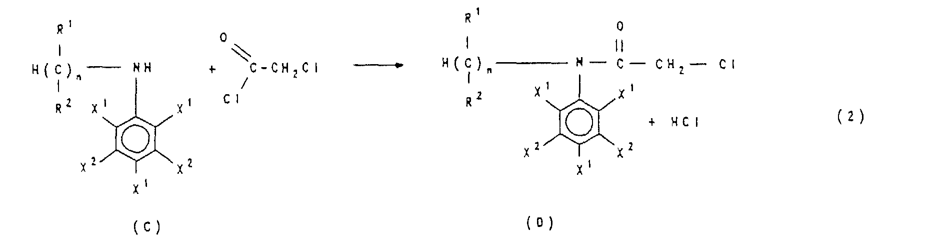 zu einer Verbindung der Formel (D) umsetzt, den gebildeten Chlorwasserstoff abtrennt, die Verbindung der Formel (D) mit einem Benzylalkohol der Formel (E), und einer Base entsprechend der Reaktionsgleichung (3) 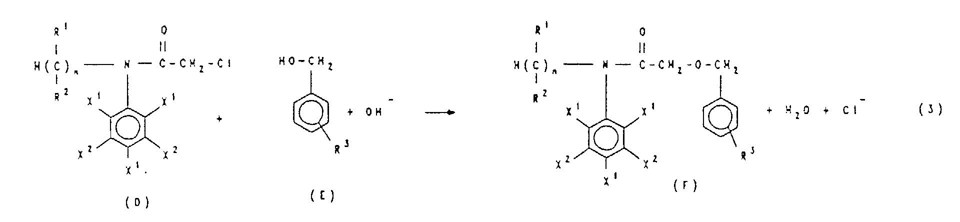 umsetzt, gegebenenfalls das aus der Base und Chlorwasserstoff gebildete Salz abtrennt, oder die Verbindung der Formel (C) mit einem O-Benzylglykoloylchlorid der Formel (K), worin R³ die in Anspruch 31 genannte Bedeutung besitzt, und gegebenenfalls mit einer Base entsprechend der Reaktionsgleichung (4) 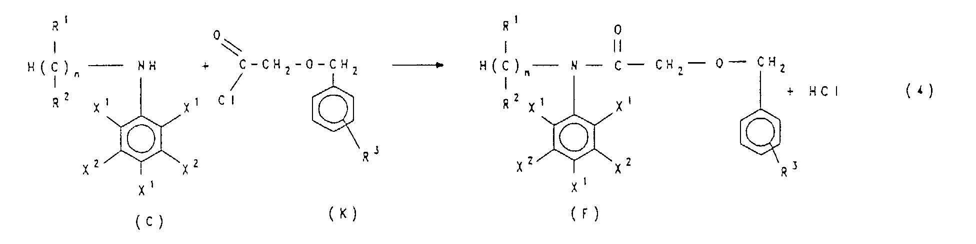 umsetzt und gegebenenfalls das aus Base und Chlorwasserstoff gebildete Salz abtrennt.

## Claims

1. A process for the preparation of glycoloylanilides of the formula (G) 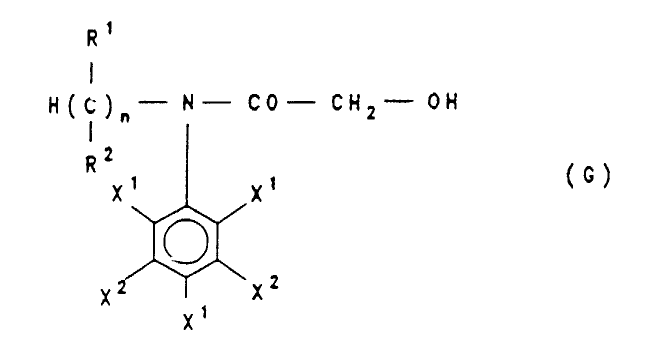 in which X¹ independently at each occurrence is H, halogen, cyano, trifluoromethyl, alkyl or alkoxy having in each case 1 to 4 carbon atoms in the alkyl moiety, and X² independently at each occurrence is H, halogen, cyano, carboxyl, trifluoromethyl, substituted or unsubstituted aminocarbonyl or aminosulfonyl, alkyl, alkoxy or alkoxycarbonyl having in each case 1 to 4 carbon atoms in the alkyl moiety, n is 0 or 1, and R¹ and R² are H, alkyl, hydroxyalkyl, alkoxyalkyl or acyloxyalkyl having in each case 1 to 4 carbon atoms in the alkyl moiety, which comprises reacting a nitrobenzene of the formula (A) with hydrogen and, if desired, with a carbonyl compound of the formula (B) in the presence of a catalyst which comprises noble metal and of a solvent, in accordance with reaction equation (1) 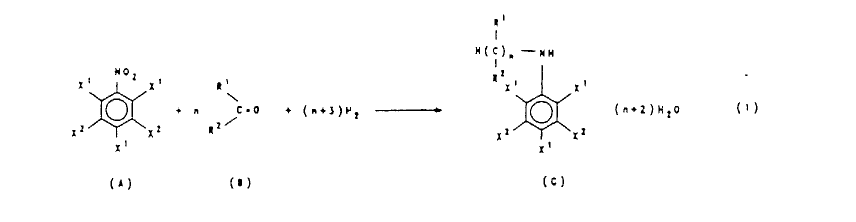 separating off the catalyst and reacting the compound of the formula (C) with chloroacetyl chloride, in accordance with reaction equation (2) 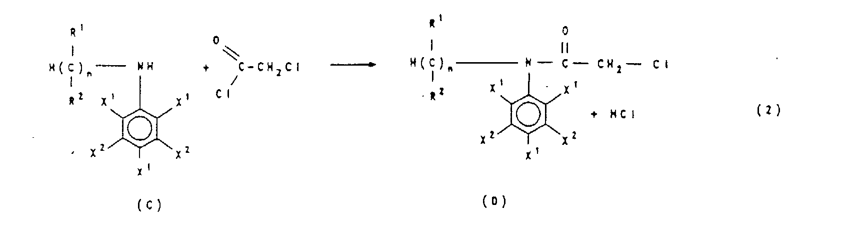 to give a compound of the formula (D), separating off the hydrogen chloride formed, reacting the compound of the formula (D) with a benzyl alcohol of the formula (E) in which R³ is H, halogen, alkyl or alkoxy having in each case 1 to 4 carbon atoms, and with a base, in accordance with reaction equation (3) 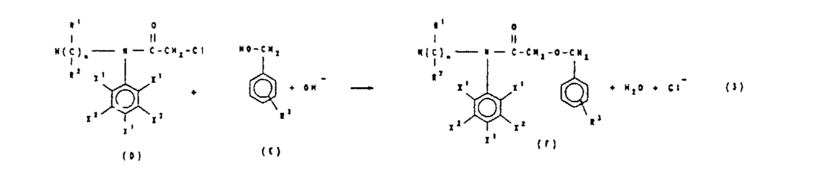 separating off if desired the salt formed from the base and hydrogen chloride, or reacting the compound of the formula (C) with an O-benzylglycoloyl chloride of the formula (K) in which R³ is as defined above, and if desired with a base, in accordance with reaction equation (4) 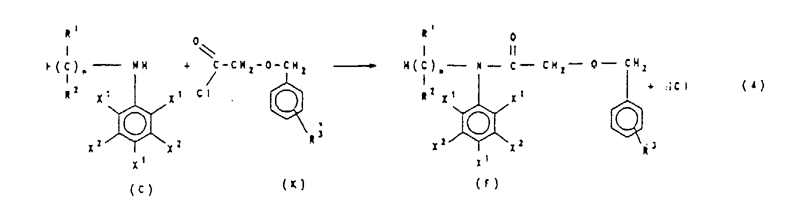 separating off if desired the salt formed from the base and hydrogen chloride, and reacting the O-benzylglycoloylanilide of the formula (F), in the presence of a catalyst which contains noble metal, with hydrogen, in accordance with reaction equation (5) 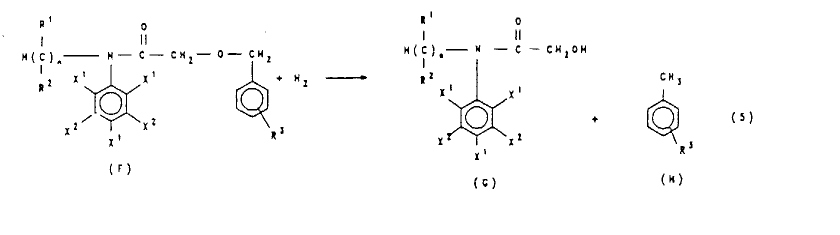 separating off the compound of the formula (H) which is formed and isolating the glycoloylanilide of the formula (G).

2. The process as claimed in claim 1, wherein a nitrobenzene of the formula (A) is employed in which X¹ independently at each occurrence is H, fluorine, chlorine or trifluoromethyl and X² independently at each occurrence is H, fluorine, chlorine, trifluoromethyl or substituted or unsubstituted aminocarbonyl.

3. The process as claimed in claim 1 or 2, wherein the nitrobenzene of the formula (A) is reacted with hydrogen at from 0.1 to 5 MPa, in particular from 0.2 to 3 MPa.

4. The process as claimed in one or more of claims 1 to 3, wherein the nitrobenzene of the formula (A) and the carbonyl compound of the formula (B) are employed in a molar ratio of 1:(1.0 to 3.5), in particular 1:(1.1 to 2.5) and preferably 1:(1.05 to 1.5).

5. The process as claimed in one or more of claims 1 to 4, wherein the catalyst, which comprises from 1 to 10% by weight, in particular from 2 to 5% by weight, of noble metal, is employed in a quantity of from 0.01 to 0.3 part, in particular from 0.025 to 0.15 part, by weight of noble metal, based on 100 parts of nitrobenzene of the formula (A).

6. The process as claimed in one or more of claims 1 to 5, wherein the catalyst which comprises noble metal employed is a supported catalyst which comprises palladium or platinum, if desired in sulfited or sulfided form, in particular an active charcoal catalyst which comprises palladium or platinum, if desired in sulfited or sulfided form.

7. The process as claimed in one or more of claims 1 to 6, wherein the solvent employed is toluene, any of the various isomeric xylenes, a mixture thereof, any of the various isomeric halotoluenes, a mixture thereof or a compound of the formula (H).

8. The process as claimed in one or more of claims 1 to 7, wherein the nitrobenzene is reacted at from 20 to 100°C, in particular from 60 to 90°C.

9. The process as claimed in one or more of claims 1 to 8, wherein the reaction mixture which comprises a compound of the formula (C) is reacted directly, without isolating the compound of the formula (C), with chloroacetyl chloride.

10. The process as claimed in one or more of claims 1 to 9, wherein the compound of the formula (C) is reacted with chloroacetyl chloride at from 0 to 150°C, in particular from 20 to 100°C.

11. The process as claimed in one or more of claims 1 to 10, wherein the compound of the formula (C) and chloroacetyl chloride are reacted in a molar ratio of 1:(1.0 to 1.5), in particular 1:(1.05 to 1.15).

12. The process as claimed in one or more of claims 1 to 11, wherein the hydrogen chloride formed is removed by boiling it off and/or by addition of a base.

13. The process as claimed in one or more of claims 1 to 12, wherein the hydrogen chloride formed is removed by boiling it off.

14. The process as claimed in one or more of claims 1 to 13, wherein the reaction mixture which comprises the compound of the formula (D) is reacted directly, without isolating the compound of the formula (D), with the benzyl alcohol of the formula (E).

15. The process as claimed in one or more of claims 1 to 14, wherein the benzyl alcohol employed is a compound of the formula (E) in which R³ is H, chlorine, methyl or methoxy.

16. The process as claimed in one or more of claims 1 to 15, wherein the compound of the formula (D) and the benzyl alcohol of the formula (E) are employed in a molar ratio of 1:(1 to 1.5), in particular 1:(1.02 to 1.2).

17. The process as claimed in one or more of claims 1 to 16, wherein the compound of the formula (D) is reacted at from 20 to 200°C, in particular from 20 to 150°C.

18. The process as claimed in one or more of claims 1 to 17, wherein the compound of the formula (D) and the base are reacted in a molar ratio of 1:(1.0 to 1.5), in particular 1:(1.02 to 1.2), with the benzyl alcohol of the formula (E).

19. The process as claimed in one or more of claims 1 to 18, wherein an alkali metal hydroxide or an alkali metal carbonate is employed as base.

20. The process as claimed in one or more of claims 1 to 8, wherein the reaction mixture which comprises the compound (C) is reacted directly, without isolating the compound of the formula (C), with an O-benzylglycoloyl chloride of the formula (K).

21. The process as claimed in one or more of claims 1 to 8 and 20, wherein an O-benzylglycoloyl chloride of the formula (K) is employed in which R³ is H, chlorine, methyl or methoxy.

22. The process as claimed in one or more of claims 1 to 8 and 20 to 21, wherein the compound of the formula (C) and the O-benzylglycoloyl chloride of the formula (K) are reacted in a molar ratio of 1:(1 to 1.15), in particular 1:(1.05 to 1.15).

23. The process as claimed in one or more of claims 1 to 8 and 20 to 22, wherein the compound of the formula (C) is reacted with the O-benzylglycoloyl chloride of the formula (K) at from 20 to 150°C, in particular from 50 to 130°C.

24. The process as claimed in one or more of claims 1 to 8 and 20 to 23, wherein the hydrogen chloride formed when the compound of the formula (C) is reacted with the O-benzylglycoloyl chloride of the formula (K) is separated by boiling it out and/or by addition of a base.

25. The process as claimed in one or more of claims 1 to 8 and 20 to 24, wherein the compound of the formula (C) is reacted with the O-benzylglycoloyl chloride of the formula (K) with the addition of an alkali metal hydroxide and/or alkali metal carbonate as base.

26. The process as claimed in one or more of claims 1 to 25, wherein the reaction mixture which comprises the O-benzylglycoloylanilide of the formula (F) is reacted with hydrogen directly, without isolating the O-benzylglycoloylanilide of the formula (F).

27. The process as claimed in one or more of claims 1 to 26, wherein a catalyst which comprises from 1 to 10% by weight, in particular from 2 to 5% by weight, of noble metal is employed in a quantity of from 0.025 to 0.5 part, in particular from 0.05 to 0.3 part, by weight of noble metal, based on 100 parts of O-benzylglycoloylanilide of the formula (F).

28. The process as claimed in one or more of claims 1 to 27, wherein the O-benzylglycoloylanilide of the formula (F) is reacted with hydrogen in the presence of a catalyst which comprises noble metal consisting of a supported catalyst comprising palladium or platinum, if desired in sulfited form.

29. The process as claimed in one or more of claims 1 to 28, wherein the O-benzylglycoloylanilide of the formula (F) is reacted at from 0.1 to 5 MPa, in particular from 0.2 to 3 MPa.

30. The process as claimed in one or more of claims 1 to 29, wherein the O-benzylglycoloylanilide of the formula (F) is reacted at from 20 to 100°C, in particular from 30 to 80°C.

31. An O-benzylglycoloylanilide of the formula (F) 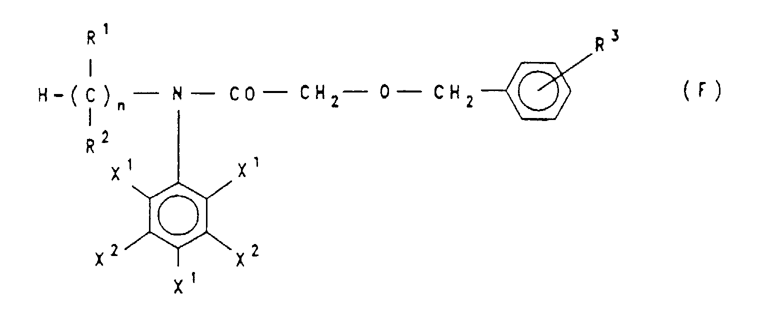 in which R¹ and R² are H, alkyl, hydroxyalkyl, alkoxyalkyl or acyloxyalkyl having in each case 1 to 4 carbon atoms in the alkyl moiety, R³ is H, halogen, alkyl or alkoxy having in each case 1 to 4 carbon atoms in the alkyl moiety, X¹ independently at each occurrence is H, halogen, cyano, trifluoromethyl, alkyl or alkoxy having in each case 1 to 4 carbon atoms in the alkyl moiety, X² independently at each occurrence is H, halogen, cyano, carboxyl, trifluoromethyl, substituted or unsubstituted aminocarbonyl or aminosulfonyl, alkyl, alkoxy or alkoxycarbonyl having in each case 1 to 4 carbon atoms in the alkyl moiety, and n is 0 or 1, in which context at least one of the radicals X² is different from H if each of the radicals X¹ is a CH₃ group, or R³ is different from H if n=0 and each of the radicals X¹ and X² is F.

32. A process for the preparation of an O-benzylglycoloylanilide of the formula (F), as indicated in claim 31, which comprises reacting a nitrobenzene of the formula (A) with hydrogen and, if desired, with a carbonyl compound of the formula (B) in the presence of a catalyst which comprises noble metal and of a solvent, in accordance with reaction equation (1) 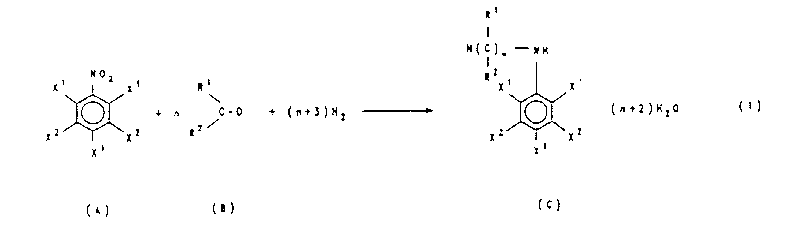 separating off the catalyst and reacting the compound of the formula (C) with chloroacetyl chloride, in accordance with reaction equation (2) 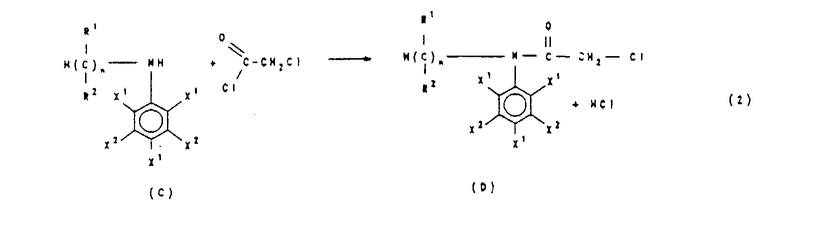 to give a compound of the formula (D), separating off the hydrogen chloride formed, reacting the compound of the formula (D) with a benzyl alcohol of the formula (E) and with a base, in accordance with reaction equation (3) 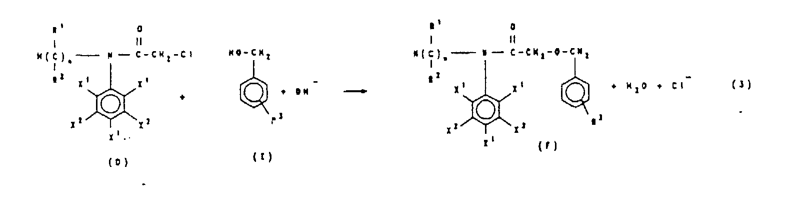 separating off if desired the salt formed from the base and hydrogen chloride, or reacting the compound of the formula (C) with an O-benzylglycoloyl chloride of the formula (K) in which R³ is as defined in claim 31, and if desired with a base, in accordance with reaction equation (4) 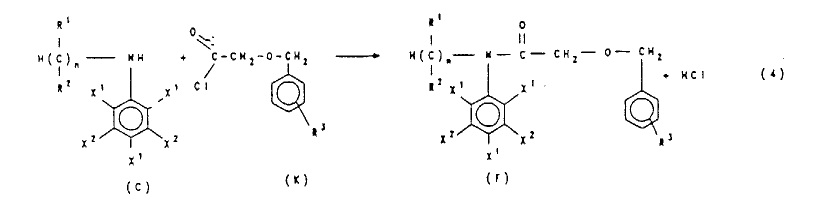 and separating off if desired the salt formed from the base and hydrogen chloride.

## Revendications

1. Procédé pour la préparation de glycoloylanilides de formule générale (G) 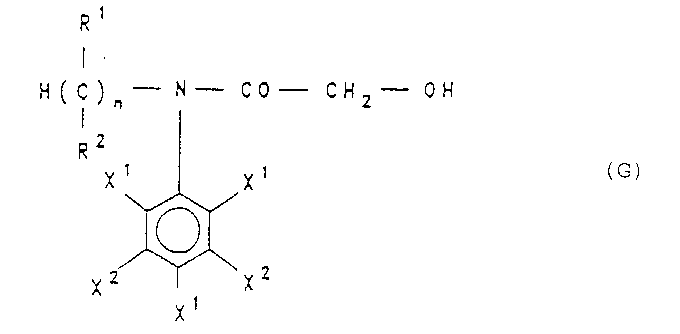 dans laquelle
X¹ représente, indépendamment, un atome d'hydrogène, un atome d'halogène, un reste cyano, trifluorométhyle, alkyle ou alkoxy, chacun avec 1 à 4 atomes de carbone dans le reste alkyle et
X² représente, indépendamment, un atome d'hydrogène, un atome d'halogène, un reste cyano, carboxyle, trifluorométhyle, un reste aminocarbonyle ou amino-sulfonyle éventuellement substitué, un reste alkyle, alkoxy ou alkoxycarbonyle, chacun avec 1 à 4 atomes de carbone dans le reste alkyle,
n vaut 0 ou 1, et
R¹ et R² représentent un atome d'hydrogène, un reste alkyle, hydroxyalkyle, alkoxyalkyle ou acyloxyalkyle, chacun avec 1 à 4 atomes de carbone dans le reste alkyle, caractérisé en ce que l'on fait réagir un nitrobenzène de formule générale (A) avec l'hydrogène et éventuellement un composé carbonylé de formule (B), en présence d'un catalyseur conntenant des métaux nobles et d'un solvant selon l'équation réactionnelle (1) 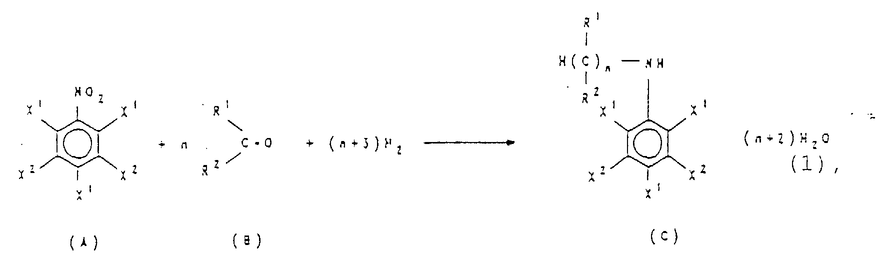
on sépare le catalyseur et on fait réagir le composé de formule (C) avec du chlorure de chloracétyle selon l'équation réactionnelle (2) 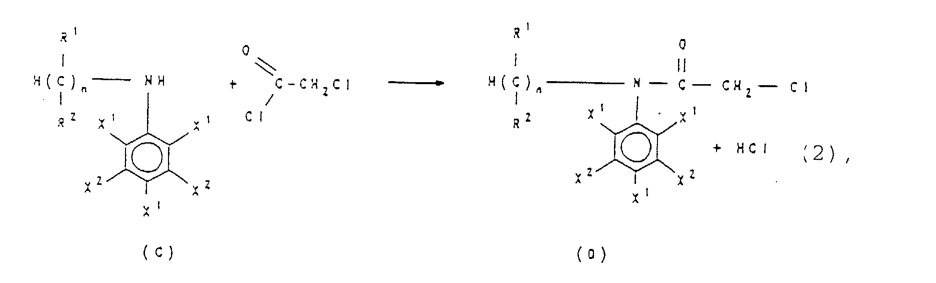 pour obtenir un composé de formule (D), on sépare le chlorure d'hydrogène formé, on fait réagir le composé de formule (D) avec un alcool benzylique de formule (E), dans laquelle R³ représente un atome d'hydrogène, un atome d'halogène, un reste alkyle ou alkoxy, chacun avec 1 à 4 atomes de carbone, et avec une base, selon l'équation réactionnelle (3) 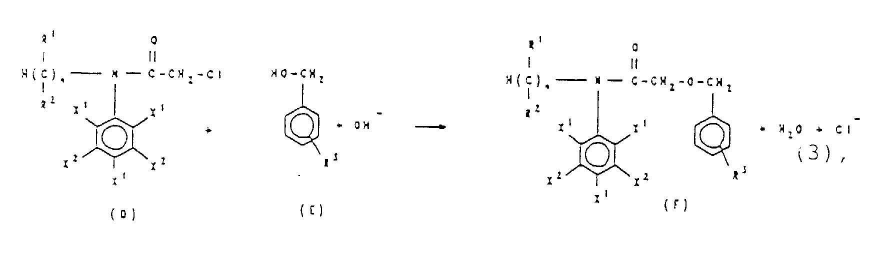 on sépare éventuellement le sel formé à partir de la base et du chlorure d'hydrogène, ou on fait réagir le composé de formule (C) avec un chlorure de O-benzylglycoloyle de formule (K), dans laquelle R³ possède la signification précitée, et éventuellement avec une base selon l'équation réactionnelle (4) 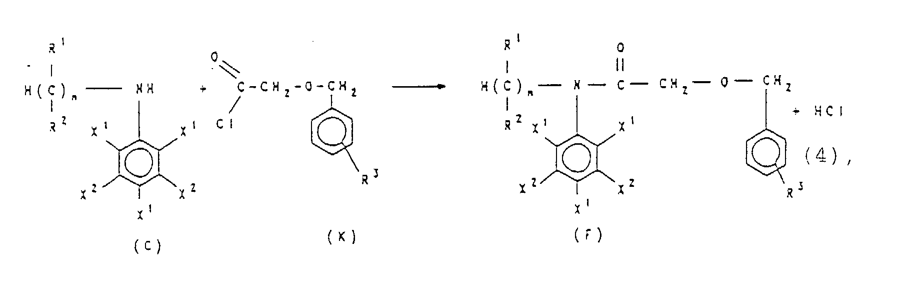 on sépare éventuellement le sel formé à partir de la base et du chlorure d'hydrogène et on fait réagir l'O-benzylglycoloylanilide de formule (F). avec l'hydrogène, en présence d'un catalyseur conntenant des métaux nobles selon l'équation réactionnelle (5) 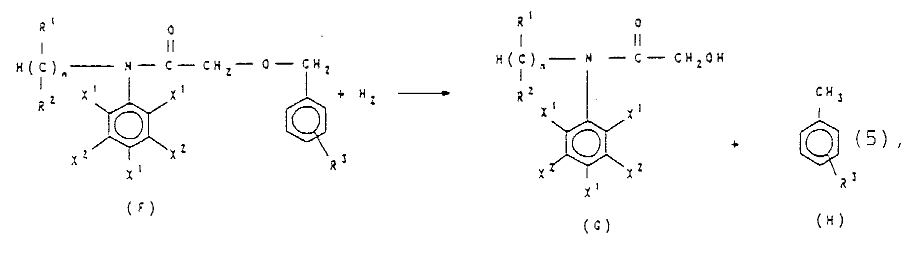 on sépare le composé de formule (H) obtenu et on isole le glycoloylanilide de formule (G).

2. Procédé selon la revendication 1, caractérisé en ce que l'on utilise un nitrobenzène de formule (A), dans laquelle X¹, indépendamment, représente un atome d'hydrogène, un atome de fluor, un atome de chlore ou un groupe trifluorméthyle et X², indépendamment, représente un atome d'hydrogène, un atome de fluor, un atome de chlore, un groupe trifluorométhyle ou un groupe aminocarbonyle éventuellement substitué.

3. Procédé selon la revendication 1 ou 2, caractérisé en ce que l'on fait réagir le nitrobenzène de formule (A) avec l'hydrogène sous une pression de 0,1 à 5, en particulier de 0,2 à 3 MPa.

4. Procédé selon une ou plusieurs des revendications 1 à 3, caractérisé en ce que l'on utilise le nitrobenzène de formule (A) et le composé carbonylé de formule (B) dans un rapport molaire de 1:(1,0 à 3,5), en particulier de 1:(1,1 à 2,5), de préférence de 1:(1,05 à 1,5).

5. Procédé selon une ou plusieurs des revendications 1 à 4, caractérisé en ce que l'on utilise le catalyseur ayant une teneur en métaux noble de 1 à 10, en particulier de 2 à 5 % en poids, dans une quantité de 0,01 à 0,3, en particulier de 0,025 à 0,15 parties en poids de métal noble, par rapport à 100 parties de nitrobenzène de formule (A).

6. Procédé selon une ou plusieurs des revendications 1 à 5, caractérisé en ce que l'on utilise en tant que catalyseur contenant le métal noble, un catalyseur supporté contenant du palladium ou du platine, éventuellement sous forme sulfurée ou sulfitée, en particulier un catalyseur sur du charbon actif contenant le palladium ou le platine, éventuellement sous forme sulfurée ou sulfitée.

7. Procédé selon une ou plusieurs des revendications 1 à 6, caractérisé en ce que l'on utilise comme solvant le toluène, les différents isomères de xylène, leurs mélanges, les différents isomères de toluènes halogénés, leurs mélanges ou le composé de formule (H).

8. Procédé selon une ou plusieurs des revendications 1 à 7, caractérisé en ce que l'on fait réagir le nitrobenzène à une température de 20 à 100, en particulier de 60 à 90 °C.

9. Procédé selon une ou plusieurs des revendications 1 à 8, caractérisé en ce que l'on fait réagir le mélange réactionnel contenant le composé de formule (C) avec le chlorure de chloracétyle directement, sans isolement du composé de formule (C).

10. Procédé selon une ou plusieurs des revendications 1 à 9, caractérisé en ce que l'on transforme le composé de formule (C) avec du chlorure de chloracétyle à une température de 0 à 150, en particulier de 20 à 100 °C.

11. Procédé selon une ou plusieurs des revendications 1 à 10, caractérisé en ce que l'on transforme le composé de formule (C) et le chlorure de chloracétyle dans un rapport molaire de 1:(1,0 à 1,5), en particulier de 1:(1,05 à 1,15).

12. Procédé selon une ou plusieurs des revendications 1 à 11, caractérisé en ce que l'on sépare le chlorure d'hydrogène formé par évaporation par ébullition et/ou ajout d'une base.

13. Procédé selon une ou plusieurs des revendications 1 à 12, caractérisé en ce que l'on sépare par évaporation par ébullition le chlorure d'hydrogène formé.

14. Procédé selon une ou plusieurs des revendications 1 à 13, caractérisé en ce que l'on fait réagir le mélange réactionnel contenant le composé de formule (D) avec l'alcool benzylique de formule (E) directement, sans isolement du composé de formule (D).

15. Procédé selon une ou plusieurs des revendications 1 à 14, caractérisé en ce que l'on utilise en tant qu'alcool benzylique un composé de formule (E), dans laquelle R³ représente un atome d'hydrogène, un atome de chlore, un groupe méthyle ou méthoxy.

16. Procédé selon une ou plusieurs des revendications 1 à 15, caractérisé en ce que l'on utilise le composé de formule (D) et l'alcool benzylique de formule (E) dans un rapport molaire de 1:(1 à 1,5), en particulier de 1:(1,02 à 1,2).

17. Procédé selon une ou plusieurs des revendications 1 à 16, caractérisé en ce que l'on transforme le composé de formule (D) à une température de 20 à 200 °C, en particulier de 20 à 150 °C.

18. Procédé selon une ou plusieurs des revendications 1 à 17, caractérisé en ce que l'on fait réagir le composé de formule (D) et la base dans un rapport molaire de 1:(1,0 à 1,5), en particulier de 1:(1,05 à 1,2), avec l'alcool benzylique de formule (E).

19. Procédé selon une ou plusieurs des revendications 1 à 18, caractérisé en ce que l'on utilise en tant que base un hydroxyde alcalin ou un carbonate alcalin.

20. Procédé selon une ou plusieurs des revendications 1 à 8, caractérisé en ce que l'on transforme le mélange réactionnel contenant le composé (C) avec un chlorure d'O-benzylglycoloyle de formule (K) directement, sans isoler le composé de formule (C).

21. Procédé selon une ou plusieurs des revendications 1 à 8 et 20, caractérisé en ce que l'on utilise un chlorure d'O-benzyglycoloyle de formule (K), dans lequel R³ représente un atome d'hydrogène, un groupe méthyle ou méthyloxy.

22. Procédé selon une ou plusieurs des revendications 1 à 8 et 20 à 21, caractérisé en ce que ce que l'on fait réagir le composé de formule (C) et le chlorure d'O-benzyglycoloyle de formule (K) dans un rapport molaire de 1:(1 à 1,15), en particulier de 1:(1,05 à 1,15).

23. Procédé selon une ou plusieurs des revendications 1 à 8 et 20 à 22, caractérisé en ce que l'on fait réagir le composé de formule (C) avec le chlorure d'O-benzyglycoloyle de formule (K) à une température de 20 à 150, en particulier de 50 à 130 °C.

24. Procédé selon une ou plusieurs des revendications 1 à 8 et 20 à 23, caractérisé en ce que l'on sépare le chlorure d'hydrogène formé au cours de la réaction du composé de formule (C) avec le chlorure d'O-benzyglycoloyle de formule (K), par ébullition et/ou par addition d'une base.

25. Procédé selon une ou plusieurs des revendications 1 à 8 et 20 à 24, caractérisé en ce que l'on fait réagir le composé de formule (C) avec le chlorure d'O-benzyglycoloyle de formule (K) sous ajout d'un hydroxyde alcalin et/ou carbonate alcalin en tant que base.

26. Procédé selon une ou plusieurs des revendications 1 à 25, caractérisé en ce que l'on fait réagir le mélange réactionnel contenant l'O-benzylglycoloylanilide de formule (F) avec l'hydrogène directement, sans isolement de l'O-benzylglycoloylanilide de formule (F).

27. Procédé selon une ou plusieurs des revendications 1 à 26, caractérisé en ce que l'on utilise le catalyseur ayant une teneur de 1 à 10, en particulier de 2 à 5 % en poids en métal noble, dans une quantité de 0,025 à 0,5, en particulier de 0,05 à 0,3 parties en poids de métal noble, par rapport à 100 parties d'O-benzylglycoloylanilide de formule (F).

28. Procédé selon une ou plusieurs des revendications 1 à 27, caractérisé en ce que l'on fait réagir l'O-benzylglycoloylanilide de formule (F) avec l'hydrogène en présence d'un catalyseur supporté contenant du palladium ou du platine, éventuellement sous forme sulfitée, en tant que catalyseur contenant des métaux nobles.

29. Procédé selon une ou plusieurs des revendications 1 à 28, caractérisé en ce que l'on fait réagir l'O-benzyglycoloylanilide de formule (F) sous une pression de 0,1 à 5, en particulier de 0,2 à 3 MPa.

30. Procédé selon une ou plusieurs des revendications 1 à 28, caractérisé en ce que l'on transforme l'O-benzyglycoloylanilide de formule (F) à une température de 20 à 100, en particulier de 30 à 80 °C.

31. O-benzyglycoloylanilides de formule générale (F) 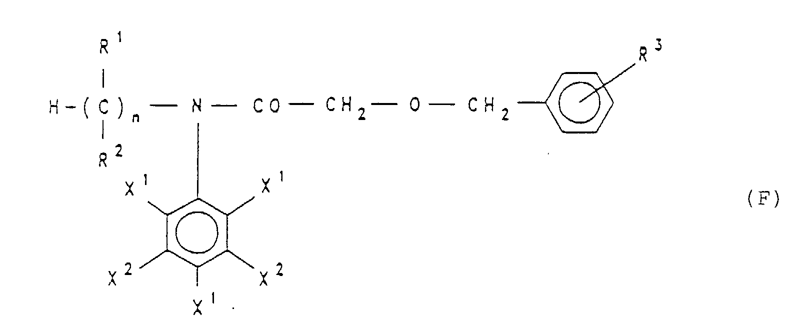 dans laquelle
R¹ et R² représentent un reste alkyle, hydroxyalkyle, alkoxyalkyle ou acyloxyalkyle, chacun avec 1 à 4 atomes de carbone dans le reste alkyle,
R³ représente un atome d'hydrogène, un atome d'halogène, un reste alkyle ou alkoxy chacun avec 1 à 4 atomes de carbone dans le reste alkyle,
X¹, indépendamment, représente un atome d'hydrogène, un atome d'halogène, un reste cyano, trifluorométhyle, alkyle ou alkoxy, chacun avec 1 à 4 atomes de carbone dans le reste alkyle,
X², indépendamment, représente un atome d'hydrogène, un atome d'halogène, un groupe cyano, carboxyle, trifluorméthyle, un reste aminocarbonyle ou amino-sulfonyle éventuellement substitué, un reste alkyle, alkoxy ou alkoxycarbonyle chacun avec 1 à 4 atomes de carbone dans le reste alkyle et
n vaut 0 ou 1,
au moins l'un des restes X² étant différent de l'hydrogène, dans le cas où chacun des restes X¹ représente un groupe CH₃, ou R³ est différent de H, dans le cas où n = 0 et chacun des restes X¹ et X² représente F.

32. Procédé de préparation des O-benzylglycoloylanilides de formule (F), comme indiqué dans la revendication 31, caractérisé en ce que l'on transforme un nitrobenzène de formule (A) avec l'hydrogène et éventuellement un composé carbonylé de formule (B), en présence d'un catalyseur contenant des métaux nobles et d'un solvant selon l'équation réactionnelle (1) 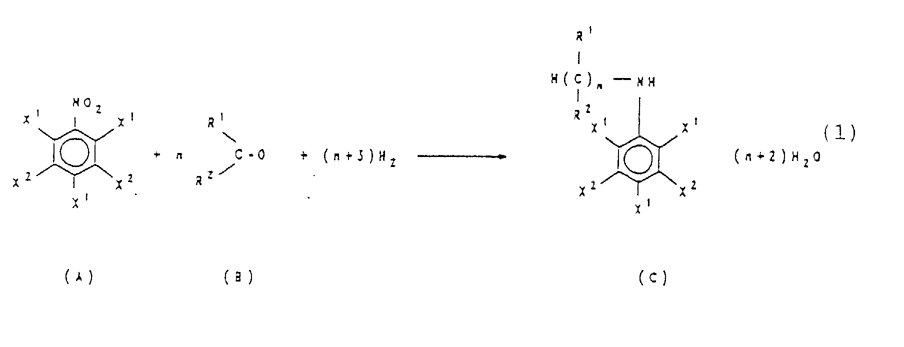 on sépare le catalyseur et on fait réagir le composé de formule (C) avec du chlorure de chloracétyle selon l'équation réactionnelle (2) 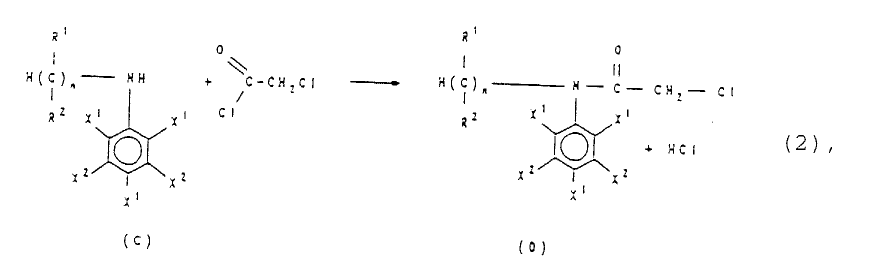 pour obtenir un composé de formule (D), on sépare le chlorure d'hydrogène formé, on fait réagir le composé de formule (D) avec un alcool benzylique de formule (E), et une base, selon l'équation réactionnelle (3) 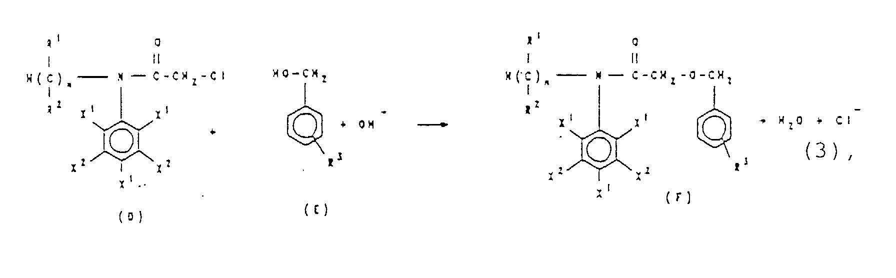 on sépare éventuellement le sel formé à partir de la base et du chlorure d'hydrogène, ou on fait réagir le composé de formule (C) avec un chlorure de O-benzylglycoloyle de formule (K), dans laquelle R³ possède la signification donnée dans la revendication 31, et éventuellement avec une base selon l'équation réactionnelle (4) 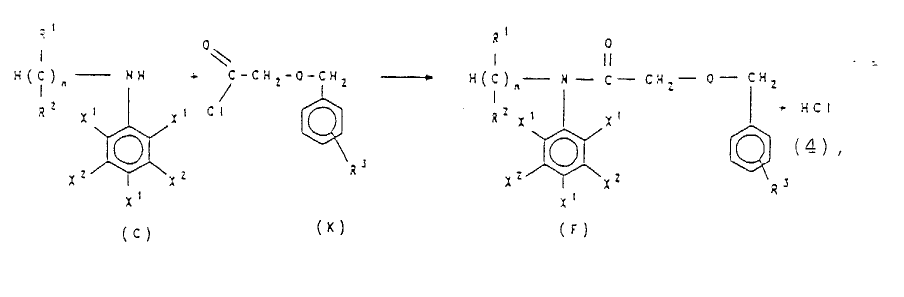 et on sépare éventuellement le sel formé à partir de la base et du chlorure d'hydrogène.
